# EUROPEAN PATENT SPECIFICATION

(11) **EP 2 717 906 B1**
(45) Date of publication and mention of the grant of the patent: **01.03.2017**
(21) Application number: 12729755.4
(22) Date of filing: 08.06.2012
(51) Int. Cl.: A61K 38/46, A61P 9/06

(54) **THE USE OF ALKALINE PHOSPHATASE FOR PRESERVING RENAL FUNCTION**
VERWENDUNG VON ALKALISCHER PHOSPHATASE ZUR AUFRECHTERHALTUNG DER NIERENFUNKTION
UTILISATION DE L'ALCALINE PHOSPHATASE POUR LA PRÉSERVATION D'UNE FONCTION RÉNALE

(30) Priority: 08.06.2011 EP 11169143
(43) Date of publication of application: 16.04.2014
(73) Proprietor: AM-Pharma B.V., 3981 AK Bunnik (NL)
(72) Inventor: AREND, Jacques Salomon Robert, NL-2312 BR Leiden (NL); VAN DEN BERG, Erik Jan, NL-5261 AC Vught (NL); RAABEN, Willem, NL-3824 NW Amersfoort (NL)
(74) Representative: V.O.
(86) International application number: PCT/NL2012/050400
(87) International publication number: WO 2012/169892

(56) References cited:
- EP-A1- 1 952 823
- EP-A1- 1 985 697
- US-A1- 2008 044 397
- JACOB SONY ET AL: "Pharmacotherapy of atrial fibrillation: a pathophysiological perspective and review.", AMERICAN JOURNAL OF THERAPEUTICS MAY 2011, vol. 18, no. 3, May 2011 (2011-05), pages 241-260, XP009165824, ISSN: 1536-3686

## Description

The invention relates to the field of medicine and in particular to means and uses in preventing and/or treating atrial fibrillation.

The kidneys perform several functions in an animal body, such as excretion of waste, acid-base homeostasis, osmolality regulation, blood pressure regulation and hormone secretion. To enable the kidneys to perform these tasks, the kidneys receive, despite their relatively small size, approximately 20% of the cardiac output. Consequently a disruption of blood flow to the kidneys (renal blood flow, RBF) has a direct impact on many of the functions of the kidney. For instance reduction of excretion of nitrogenous waste and disturbances of fluid and electrolyte balances could then occur. On the long term, reduced RBF, but also other toxic events, such as ischemia (reperfusion injury), use of contrast media or (other) nephrotoxic drugs, e.g. antibiotics, can be so stressful to the kidneys that it results in acute kidney injury (AKI).

It is very important to prevent and/or adequately treat AKI, because first, AKI is accompanied with high costs, morbidity and mortality, and second, prolonged AKI may lead to chronic kidney injury (CKI), possibly leading to irreversible loss of renal function. Additionally, an individual that has acquired CKI is more prone to (again) acquire AKI, which is then called "acute on chronic kidney injury". Because the renal function in CKI patients is already reduced, acute on chronic kidney injury may reduce renal function below a critical threshold. It is therefore that prevention of AKI in CKI patients is particularly important. It has further been speculated that AKI on top of CKI leaves the patient with an even more reduced kidney function, even after the acute component of the kidney injury is resolved.

Depending on the cause of AKI (pre-renal, renal or post-renal), different treatment methods are available. If for instance reduced RBF is causative for AKI, early RBF normalization predicts better prognosis for recovery of renal function. Initial treatment should focus on correcting fluid and electrolyte balances and uraemia while the cause of acute AKI is being sought. A volume-depleted patient is resuscitated with saline. More often, however, volume overload is present, especially if patients are oliguric or anuric.

If AKI is not caused by a reduction in RBF, but for instance results from ischemia or exposure to nephrotoxic agents, such as contrast agents or antibiotics, it is important to take away this cause of AKI. This can for instance be done by removing the cause of ischemia, for instance a thrombus, or by stopping kidney damaging medication.

One example of a current treatment of AKI caused by reduced RBF is intravenous administered Furosemide (Lasix). Another example of one of the current treatments is intravenously administered calcium. Potassium can be temporarily shifted into the intracellular compartment using intravenously administered insulin (10 units) and glucose (25 g), inhaled beta agonists or intravenously administered sodium bicarbonate. Potassium excretion is achieved with sodium polystyrene sulphonate (Kayexalate) and/or diuretics. Sodium polystyrene sulphonate is given orally (25 to 50 g mixed with 100 mL of 20 percent sorbitol) or as an enema (50 g in 50 mL of 70 percent sorbitol and 150 mL of tap water). If these measures do not control the potassium level, dialysis is initiated.

Acidosis is typically treated with intravenously or orally administered sodium bicarbonate if the serum bicarbonate level is less than 15 mEq per L (15 mmol per L) or the pH is less than 7.2. Patients can also be treated orally with sodium bicarbonate tablets, Shohl's solution in 30-mL doses or powdered sodium bicarbonate. Serum bicarbonate levels and pH should be followed closely. Intractable acidosis requires dialysis.

All medications should be reviewed, and their dosages should be adjusted based on the glomerular filtration rate and the serum levels of medications.

Between 20 and 60 percent of AKI patients require short-term dialysis, particularly when the blood urea nitrogen (BUN) exceeds 100 mg per dL (35.7 mmol per L of urea) and the serum creatinine level exceeds the range of 5 to 10 mg per dL (442 to 884 µmol per L). Indications for dialysis include acidosis or electrolyte disturbances that do not respond to pharmacologic therapy, fluid overload that does not respond to diuretics, and uraemia. The EMEA for instance, advises the following criteria, which are in line with ADQII recommendations for determining RRT requirement:
a) oliguria < 0.3ml/kg/h OR
b) anuria for > 12h AND serum urea > 15mmol/L OR
c) hyperkalemia > 7.5 mmol/L or with typical ECG changes despite therapeutic intervention OR
d) acidosis with standard bicarbonates < 15 mmol/L despite therapeutic intervention OR
e) hyponatremia < 120mmol/L despite therapeutic intervention OR
f) clinical diagnosis of fluid overload with established or threatening pulmonary or brain oedema despite therapeutic intervention.

In some cases patients suffering from CKI and/or AKI are unable to receive an important medical treatment for a(nother) condition, because of the possible toxic effects such treatment can have. For instance a threshold for creatinine level in the blood could be set at 1.4 mg/dL in order to receive iodinated contrast. Although seemingly contradictory, under certain circumstances renal replacement therapy (RRT) itself is also stressful for the kidneys.

Normally, RRT depletes volume from within the intravascular space because, as a result of waste removal, fluids are also removed. The depleted volume is slowly recruited from the extravascular space. However, in critically ill patients, which are already hypovolemic, the depletion of intravascular volume may lead to ischemic insults, for instance in the kidneys. It is therefore that RRT is considered a treatment stressful for the kidneys. It has also been reported that patients that require chronic RRT over time (3-12 months) may lose any residual renal function they may have, probably as a result of RRT.

It is therefore that alternative means and/or methods are needed that preserve and or increase renal function, prevent or shorten duration of RRT, or increase kidney creatinine clearance.

A goal of the present invention is to provide means and methods to prevent or treat atrial fibrillation.

Pharmakotherapy of atrial fibrillation is reviewed in Jacob et al, Am. J. of Therapeutics 18, 241-260 (2011).

The present disclosure provides alkaline phosphatase (AP) for use in at least in part preserving and/or increasing renal function after a treatment with a risk of reducing renal function. Such treatment involves administering said alkaline phosphatase prior to, and optionally during and/or after the treatment with a risk of reducing renal function. Also disclosed is the use of alkaline phosphatase for the manufacture of a medicament for at least in part preserving and/or increasing renal function after a treatment with a risk of reducing renal function. Said alkaline phosphatase is preferably administered prior to, and optionally during and/or after the treatment with a risk of reducing renal function.

As used herein, the term "preserving" includes preventing a reduction, slowing down a reduction, stopping a reduction and/or at least partly reversing a reduction of a renal function. The term "increasing" is not necessarily limited to increasing said renal function to a value equal to or higher than that before said treatment occurred. It includes partly restoring renal function.

The term "treatment with a risk of decreasing renal function" is typically used to refer to a treatment which bears the risk that renal function is reduced by said treatment when comparing the value of at least one renal related parameter to a recognised or average (laboratory) value of said parameter, or by comparing said parameter to the value before said treatment is performed. If, for example, the amount of protein in the urine of a subject, preferably a human being is significantly above a recognised or average (laboratory) value, said renal function is said to be "decreased". The corresponding analysis can be performed in a laboratory but also in a home setting. For example, since September 2006 the Dutch "Nierstichting" has introduced a simple test (named Kidney check ("Niercheck")) which can be performed at home to test whether the kidneys function properly. This test is for instance directed to the amount of protein in the urine.

Other examples of parameters that can be reviewed in order to determine renal function are glomerular filtration rate (GFR), endogenous creatinine clearance as an approximation of GFR, serum creatinine levels, electrolyte derangement, amount of produced urine, BUN, calcium levels, phosphorous levels, albumin levels, and/or red and white blood cells in urine. Other tests that can be performed are a complete blood count with differential.

As disclosed herein within the experimental part it is also possible to analyse a urine sample on the presence of absence of an RNA molecule. Preferably, said RNA molecule is an mRNA molecule. Even more preferred, said mRNA molecule is iNOS mRNA. In a most preferred embodiment said RNA is obtained from urine-secreted renal cells.

A treatment with a risk of reducing renal function thus includes any treatment that has the potential to negatively influence any of the above mentioned parameters, such that renal function is said to be decreased. Typical treatments that may carry a risk of reducing renal function are renal replacement therapy, exposure to intravascular contrast media, kidney and/or heart surgery, kidney and/or heart transplantation, blood transfusion, red blood cell transfusion, nephrotoxic drug treatment and/or surgical re-exploration. The present disclosure provides the insight that AP is very well capable of preventing kidney damage when such treatment carrying a risk of reducing renal function is performed. It is preferred that AP is administered before the treatment carrying the risk of reducing renal function is performed, in order to prevent reduction in kidney function.

This new insight is especially useful for a subject already confirmed as having a reduced renal function and scheduled for a treatment with a risk of reducing renal function. In general, such treatment has a risk of reducing renal function even further. This may be problematic if the subject scheduled for such treatment has only little renal function left. In such case it may be necessary to postpone or even refrain from such treatment, such as e.g. administration of contrast media for diagnostic purposes. This is of course not an ideal situation as such treatment or diagnostic method may be important for the patients well being. Up to the present disclosure, it had to be decided whether to refrain from the treatment or take the risk of reducing renal function even further. However, with the insight provided by the present disclosure it is no longer necessary to choose between these two options, as AP is able to preserve renal function in a person undergoing a treatment having a risk of reducing renal function. Therefore, the disclosure provides a method for at least in part preserving and/or increasing renal creatinine clearance in a subject undergoing a treatment with a risk of decreasing renal creatinine clearance, thereby at least in part preserving and/or increasing said renal creatinine clearance. This is particular useful if such person undergoing such treatment has a risk of reduction of renal function below a critical threshold. According to the present disclosure, alkaline phosphatase is able to prevent reduction of the renal function below a critical threshold and thus enables such person to receive the treatment, now carrying a much lower risk of reducing renal function. Preferably a renal function, preferably the renal creatinine clearance, is determined before AP is used for preserving renal function in order to determine the risk that the renal function of said person is reduced below a certain threshold level.

Preferably, a use of AP according to the disclosure is provided, wherein said AP is for administration to a subject already suffering from renal disease. As said above, such person, already suffering from renal disease bears a higher risk that a treatment carrying a risk of reducing renal function results in a renal function below a threshold level. Preferably AP is used according to the invention in a subject already suffering from renal disease which has an estimated or calculated GFR of less than about 75 ml/min per 1.73 m², more preferably less than about 55 ml/min per 1.73 m², more preferably less than about 45 ml/min per 1.73 m², most preferably less than about 35 ml/min per 1.73 m². Especially at these low GFR values, it is very important to preserve the remaining renal function. Therefore, it is important that persons having such low GFR values receive AP in order to preserve as much renal function as possible. GFR can be assessed in several ways: Firstly it can be measured by Inulin or Chromium EDTA clearance. This is, however, restricted to experimental settings, and in general too costly and time consuming for general clinical practice. Alternatively, an approximation of GFR can be made, for instance by calculating the endogenous creatinine clearance (ECC). This is calculated from a measured 24 hour urine volume, the urine creatinine level and the serum creatinine level. This is not routinely done in clinical practice, but in principle it is possible. In this ECC calculation there are systematic errors, and results tend to overestimate the actual GFR. The other methods for assessing GFR are estimation methods. there are two main methods known in the art, i.e. Cockroft-Gault and MDRD, both are more reliable in chronic kidney disease (CKD) than in AKI.

Although short term preservation of renal function can already have immediate life-saving consequences, it is preferred that the effect of AP on the renal function is long lasting.

It is preferred that an AP is used to preserve renal function after a treatment with a risk of reducing renal function, wherein said treatment is of relative short duration. It is preferred that said treatment is less than about 4 days. When said treatment comprises for instance iodinated contrast media, such treatment is relative short. One advantage of using AP for such short treatments is that AP is also administered for a relative short period which for instance enhances medication compliance.

AP for preserving a renal function after a treatment with a risk of reducing renal function is disclosed herein, wherein the duration of said treatment with a risk of reducing renal function is less than 4 days, preferably less than 3 days, more preferably less than 2 days, most preferably less than 1 day.

As already said before, the kidney has several functions. One very important function is the clearance of nitrogenous waste products from the blood. If these nitrogenous waste products are not cleared by the kidney, they build up in the body and can lead to toxicity, e.g. in the central nervous system. The clearance function of the kidneys is preferably measured by renal (endogenous) creatinine clearance, but for instance other forms of estimating the GFR are also suitable. Creatinine clearance can be determined by methods known in the art. One method of calculation of endogenous creatinine clearance is to collect urine (usually for 24-hours) to determine the amount of creatinine that was removed from the blood over a given time interval. If one removes, for instance, 1440 mg in 24 hours, this is equivalent to removing 1 mg/min. If the blood concentration is 0.01 mg/mL (1 mg/dL), then the creatinine clearance is said to be 100 mL/min. This is because, in order to excrete 1 mg of creatinine, 100 mL of blood containing 0.01 mg/mL would need to have been cleared. According to the disclosure, AP is very well capable of retaining renal function. As a consequence AP is capable of improving many renal function related parameters, such as BUN, creatinine clearance and serum creatinine levels. It is especially useful to preserve the creatinine clearance, as reduced creatinine clearance generally results in increase of creatinine and other toxic waste products in the serum.

A use of AP is disclosed herein, wherein said preserving and/or increasing renal function comprises preserving and/or increasing renal creatinine clearance relative to the renal creatinine clearance before said treatment with a risk of reducing renal function.

As said before, a treatment with a risk of reducing renal function can be any treatment that has the potential of decreasing renal function in a subject when said subject has received said treatment. Such treatment comprises for instance renal replacement therapy (RRT), exposure to intravascular contrast media, kidney and/or heart surgery, kidney and/or heart transplantation, blood transfusion, red blood cell transfusion, drug treatment and/or surgical re-exploration. The disclosure now provides the insight that AP is suitable for preserving renal function during all these different treatments.

As already explained above, reduced renal function can be an indication for RRT, but in some cases reduced renal function may also be a contraindication for RRT. This is explained by the fact that RRT generally is accompanied by intravasal fluid depletion and critically ill patients do not tolerate fluctuations in amount of intravasal fluid. This may ultimately lead to hypovolemic shock, which compromises the blood flow to the kidneys, exposing the kidneys to an ischemic insult. As already mentioned above, complete loss of residual renal function has been observed in subjects receiving chronic RRT. The present disclosure now provides the insight that such complete loss of residual renal function in subjects receiving chronic RRT can be prevented when AP is administered before and/or during RRT. Therefore, the disclosure provides the use of AP for preventing and/or decreasing a loss of renal function in a subject receiving chronic RRT.

Another treatment with a risk of reducing renal function is the use of intravascular contrast media, such as for instance iodinated contrast media. Especially the ionic contrast media, which are highly hyperosmolar in comparison with (blood)plasma are often associated with a reduction in renal function. Without being bound to theory, the effect of ionic contrast media may be explained as follows. The hyperosmolar contrast agent may attract extravasal fluids into the vascular system, which can cause the arteries of the kidneys to expand. When the arteries expand vasoconstrictors are released to compensate for the artery expansion. These actions may result in a rapid opening and closing action of the arteries. The result of this action is a diminished blood supply to kidneys which can lead to total shut down of the kidneys. There is also a chance that the arteries may be constricted to an extent that they totally close. The body attempts to regulate the fluid overload in the vascular system. However, if the kidneys are non-functional, either as a result of the contrast agent or independent thereof, the fluid is forced to seek other avenues of escape causing fluid overload to occur in other body systems. One of the major results of this event is pulmonary oedema. According to the disclosure, AP is used to retain or improve renal function when ionic contrast media are used. In such case, a patient requiring contrast media can receive such necessary treatment with reduced risk of reducing renal function. In cases were, before the present disclosure, contrast media were contra-indicated in patients with (severe) reduced renal function, it is now possible to treat such patients with AP in order to enable them to receive such contrast media.

It has been shown that cardiac surgery can also reduce renal function and that not only cardiac surgery itself is a risk factor for AKI, but also that peri-operative parameters influenced independently the risk on AKI after cardiac surgery. These parameters were: preoperative anaemia, red blood cell transfusion and surgical re-exploration. Without being bound to theory, these parameters, and heart surgery as such, are all believed to cause ischemia to the kidney. Of course, not only heart surgery, but also for instance kidney surgery or transplantation causes ischemia to renal tissue, which is therefore also regarded as a treatment with a risk of reducing renal function. As cardiac surgery is a treatment carrying a risk of reducing renal function, administration of AP is capable of improving or retaining renal function in a patient receiving cardiac surgery.

Other treatments having a risk of reducing renal function include for instance the use of certain medication or drugs. Renal dysfunction and injury secondary to medications are common, and can present as subtle injury and/or overt renal failure. Some drugs perturb renal perfusion and induce loss of filtration capacity. Others directly injure vascular, tubular, glomerular and interstitial cells, such that specific loss of renal function leads to clinical findings, including microangiopathy, Fanconi syndrome, acute tubular necrosis, acute interstitial nephritis, nephrotic syndrome, obstruction, nephrogenic diabetes insipidus, electrolyte abnormalities and chronic renal failure. Specifically, the use of certain anti-microbial agents (such as Amphotericin B, caspofungin, vancomycin, levofloxacin, and aminoglycosides such as tobramycin and gentamicin), other drugs (e.g. chemotherapeutic agents (such as cisplatin, carboplatin, methotrexate), protease inhibitors (such as indinavir and ritonavir), gold, lithium, anti-inflammatory drugs (such as non-steroidal anti-inflammatory drugs, cyclosporin, tacrolimus, sirolimus), anti hypertensive drugs (such as angiotensin converting enzyme (ACE) inhibitors and angiotensin receptor blockers (ARBs)) and certain chemicals (such as silicates, hydrocarbons, heavy metals (such as Cd, Hg, Pb), insecticides, herbicides, ethylene glycol and bacterial toxins (such as tetanus, streptococcal toxins)) are known to bear the risk of reducing renal function in subjects who have taken or have been exposed to said agents or chemicals. According to the present disclosure, it is now possible to reduce the effect of these agents or chemicals by administering AP to subjects exposed to such agents or chemicals. It also possible to reduce the amount of certain drugs, as the present invention provides the insight that a use of AP according to the invention reduces the amount of co-medication in a patient.

A use of AP is disclosed, wherein said treatment with a risk of reducing renal function comprises renal replacement therapy (RRT), exposure to intravascular contrast media, kidney and/or heart surgery, kidney and/or heart transplantation, blood transfusion, red blood cell transfusion, drug treatment, and surgical re-exploration.

A treatment having a risk of reducing renal function may result in reducing the blood flow through the kidneys. The outer medulla of the kidney is especially susceptible to injury and/or cell damage when the blood supply is decreased, leading to an ischemic event. According to the disclosure, it is especially useful to use AP for retaining or improving renal function when the mean arterial blood pressure is, or is expected to become, less than 80 mm Hg. Below a mean arterial blood pressure of 80 mm Hg, the kidney is at risk of being injured, which generally results in a reduction of renal function.

Therefore, a use of AP is disclosed, wherein said treatment with a risk of reducing renal function results in or is accompanied by, hypoperfusion of the kidney, preferably due to mean arterial blood pressure being below 80 mm Hg, preferably below 75 mm Hg, more preferably below 70 mm Hg, more preferably below 65 mm Hg, most preferably below 60 mm Hg. According to the disclosure, AP is able to, at least in part, prevent a reduction in renal function by preventing kidney injury due to the drop in mean arterial blood pressure.

AP is preferably used in order to preserve and/or improve renal function during a treatment having a risk of reducing renal function, because a transient decrease in renal function often results in a condition which in itself is possibly damaging for the kidney, such as for instance RRT requirement. As said before, RRT itself may be contraindicated in subjects having reduced renal function. It is therefore, that in a use of AP according to the disclosure is provided, wherein said renal function is preserved during said treatment with a risk of reducing renal function. In such case, RRT requirement is preferably prevented. This is especially useful in subjects which already have a (severe) reduction in renal function.

Preferably, an alkaline phosphatase is used for shortening the duration of RRT. Thus, an alkaline phosphatase for use in a method for shortening the duration of RRT through at least in part preserving and/or increasing renal function during and/or after RRT, by administering said AP prior to, and optionally during and/or after RRT is disclosed herein. As said before, RRT and especially chronic RRT is considered a treatment with a risk of reducing renal function. It is therefore that it is especially useful to shorten the duration of RRT in order to decrease the risk of reducing renal function. RRT is preferably stopped when a renal function has reached a threshold level, such that RRT is no longer required. Therefore, a use of AP is disclosed, wherein a renal function, preferably GFR, more preferably renal creatinine clearance is determined and said RRT is stopped when said renal function, preferably said GFR, more preferably said renal creatinine clearance, is increased to and/or preserved at a threshold level.

As already said before, it is preferred that the preservation and/or increase in renal function, such as kidney creatinine clearance, is long lasting. However, it is also preferred that such preservation and/or increase already starts during a treatment carrying a risk of reducing renal function, such as RRT. It is thus preferred that use of AP preserves and/or increases kidney creatinine clearance during RRT. In a working example, the present disclosure shows that alkaline phosphatase is able to preserve and/or increase kidney creatinine clearance after RRT.

A critical creatinine clearance level, in particular for chronic kidney disease, is considered to be about 15-25 ml/min. Below said threshold, nitrogenous waste builds up in the body, possibly exerting toxic effects. It is therefore that, a use of alkaline phosphatase for increasing and/or preserving kidney creatinine clearance at a level of at least 15 ml/min, preferably at least 20 ml/min, more preferably at least 25 ml/min, most preferably at least 50 ml/min is disclosed herein.

The disclosure furthermore provides the insight that patients in ICU required significantly less medication when AP was administered. In a working example it was for instance shown that especially medication that was given for the treatment of a heart condition, such as a heart condition resulting from myocardial infarction or heart failure, was administered significantly less to subjects that received AP in comparison to subjects receiving placebo medication. Now that the disclosure provides this insight, it is possible to use AP for reducing the amount of medication for the treatment of a heart condition, thereby reducing for instance the adverse side effects of such medication.
Therefore, us of an alkaline phosphatase is disclosed, for reducing the amount of (co-)medication, thereby at least in part preserving and/or increasing renal function. Further, the disclosure provides an alkaline phosphatase for use in a method for reducing the amount of (co)medication in a patient already suffering (or at risk of suffering) from reduced renal function.
Preferably, said (co)medication is for the treatment of a heart condition, preferably a heart condition resulting from myocardial infarction or heart failure. Preferably, said (co)medication is a diuretic medicament. Diuretic medicaments influence the fluid balance in an individual by forced diuresis. Diuretics have been proven useful for, amongst others, the treatment of heart failure, hypertension and renal diseases. On the other hand, in a person already suffering from reduced renal function, diuretics may reduce renal function even further, as a result of the reduction in blood pressure due to diuresis. AP, however, is able to reduce such reduction in blood pressure and/or prevent or improve reduced renal function resulting from such reduction in blood pressure.
Preferably, use of an alkaline phosphatase according to the invention for reducing (co-)medication is disclosed, wherein the (co)medication is a class III anti-arrhythmic, a selective β-blocker, a digitalis glycoside, a vitamin K antagonist, or a selective β-2-adrenoceptor agonist. In a working example, it has been shown that the need for such medication is reduced in subjects receiving AP.
Preferably, a use of alkaline phosphatase for reducing (co-)medication is disclosed, wherein the (co)medication is potassium, a benzo-derivative, a plain sulfonamide, magnesium and/or digitalis glycosides. As the above mentioned drugs are potentially harmful for the kidney, the use of AP for reducing the amount of these drugs, consequently reduces the risk of reducing renal function in patients receiving AP.
Without being bound to theory, it is believed that the reduction in (co)medication observed in individuals that received AP is a direct or indirect beneficial effect of AP on the risk of atrial fibrillation or other cardio- or vascular disorders. In one embodiment, therefore, a use of AP according to the invention is provided, wherein said AP is for use in a method for preventing and/or treating atrial fibrillation, wherein said atrial fibrillation is due to reduced renal function.
In one embodiment, therefore, the invention provides an alkaline phosphatase for use in a method for preventing and/or treating atrial fibrillation, wherein said atrial fibrillation is due to reduced renal function. Also the use of AP for the preparation of a medicament for any of the above indications is disclosed.

Now that the disclosure has provided the insight that AP is useful for preserving and/or increasing renal function in an individual undergoing a treatment with a risk of reducing renal function, methods are disclosed for the treatment of individuals at risk of renal function reduction.

A method is disclosed for at least in part preserving and/or increasing GFR, preferably renal creatinine clearance in a subject undergoing a treatment with a risk of reducing GFR and/or renal creatinine clearance, the method comprising
administering alkaline phosphatase before, and optionally during and/or after said treatment, thereby at least in part preserving and/or increasing said GFR, preferably said renal creatinine clearance and
determining said preserved and/or increased GFR, preferably said renal creatinine clearance at least 7 days after, preferably at least 14 days after, more preferably at least 21 days after, most preferably at least 28 days after said treatment with a risk of reducing renal creatinine clearance.

It is preferred that shortly after the start of administering AP, a decision is made whether AP treatment is continued or stopped. Individuals receiving AP for renal function preservation are preferably divided in subjects which respond well to AP treatment and subjects which do not respond well to AP treatment. It is preferred that good responders receive continuous administration of AP until a threshold level of renal function is achieved, whereas it is preferred that subjects which do not respond well do not necessarily receive further AP medication. Subjects which do not respond well to AP treatment are preferably switched to another kind of medication such as diuretics or other medication supportive for renal function. Therefore, a method is disclosed for determining whether a subject undergoing a treatment with a risk of reducing GFR and/or renal creatinine clearance responds well to an alkaline phosphatase treatment for at least in part preserving and/or increasing GFR, preferably renal creatinine clearance, the method comprising administering alkaline phosphatase before, and optionally during and/or after said treatment;
determining GFR, preferably renal creatinine clearance at baseline and/or on the first day after the start of alkaline phosphatase administration;
determining GFR, preferably renal creatinine clearance on the second and/or third day after the start of alkaline phosphatase administration, wherein an increase of said GFR and/or said renal creatinine clearance on the second and/or third day, relative to said GFR and/or renal creatinine clearance at baseline and/or on the first day is indicative for said subject responding well to said alkaline phosphatase treatment; and
determining whether said individual responds well to said alkaline phosphatase treatment.
It is not only possible to discriminate between subjects responding well to AP and subjects which do not respond well to AP by comparison of a renal function at baseline and/or on day 1 with a renal function on the second and/or third day, but also by comparing a renal function on the second and/or third day after the start of alkaline phosphatase administration with a threshold level. Such threshold level is set at for instance a creatinine clearance of 15 ml/min, preferably 20 ml/min, more preferably 25 ml/min, more preferably 50 ml/min, most preferably 75 ml/min. Such threshold may depend for instance on the general condition of the subject in question or for instance the renal function at baseline, i.e. before AP is administered.
Therefore, a method is disclosed for determining whether a subject undergoing a treatment with a risk of reducing GFR and/or renal creatinine clearance responds well to an alkaline phosphatase treatment for at least in part preserving and/or increasing GFR, preferably renal creatinine clearance, the method comprising administering alkaline phosphatase before, during and/or after said treatment;
determining GFR, preferably renal creatinine clearance on the second and/or third day after the start of alkaline phosphatase administration, wherein a renal creatinine clearance of at least 15 ml/min, preferably at least 20 ml/min, more preferably at least 25 ml/min, more preferably at least 50 ml/min, more preferably at least 75 ml/min on the second or third day is indicative for said subject responding well to said alkaline phosphatase treatment; and
determining whether said individual responds well to said alkaline phosphatase treatment.
A comparable threshold for GFR can easily be determined by the skilled person based on the creatinine clearance thresholds given above. A person skilled in the art is able to estimate GFR on the basis of a given creatinine clearance and vice versa. The skilled person may be guided for instance by Levey et al.
As said before, alkaline phosphatase is preferably administered to a subject receiving a treatment with a risk of reducing renal function, wherein the duration of said treatment is relatively short. Preferably, for such short treatment, AP is administered for a short period as well, thus improving for instance medication compliance. Therefore, a method is disclosed, wherein the duration of said treatment with a risk of reducing renal function is less than 4 days, preferably less than 3 days, more preferably less than 2 days, most preferably less than 1 day. As said before, AP is especially useful for treating patients already suffering from reduced renal function, because such patients are prone to have their renal function reduced below a threshold level, which may result in AKI. In order to determine whether an individual undergoing a treatment with a risk of reduced renal function is already suffering from reduced renal function, said renal function is preferably determined before starting said potentially kidney damaging treatment. Therefore, a method according to the description is disclosed, said method further comprising determining glomerular filtration rate, creatinine clearance, and/or urinary KIM-1 levels before starting said treatment, as discussed herein before. Preferably, said treatment with a risk of reducing renal function comprises renal replacement therapy, exposure to intravascular contrast media, kidney and/or heart transplantation, kidney and/or heart surgery, blood transfusion, red blood cell transfusion, and/or surgical re-exploration. Preferably, AP is used during treatment that would result in, or would be accompanied by, hypoperfusion of the kidney, preferably due to mean arterial blood pressure being below 80 mm Hg, preferably below 75 mm Hg, more preferably below 70 mm Hg, more preferably below 65 mm Hg, most preferably below 60 mm Hg if AP would not have been administered. Such low arterial blood pressure generally results in reduced renal function and/or kidney injury. Use of AP during said treatment, however, results in less hypoperfusion, less reduction in renal function and/or less kidney damage.

As said before, AP is especially useful for shortening RRT. Therefore, a method for shortening duration of RRT in a subject undergoing RRT is disclosed, the method comprising
administering alkaline phosphatase before, during and/or after said subject undergoes RRT; and
determining renal function before, during and/or after said subject undergoes RRT; and
discontinuing RRT in said subject when said renal function has been increased to and/or preserved at a threshold value, thereby shortening duration of RRT in said subject. As said before, reduction of RRT is especially useful as (chronic) RRT is considered a treatment with a risk of reducing renal function. As said before, AP is especially useful in reducing the amount and/or adverse side effects of (co-)medication. Also disclosed, therefore, is a method for
decreasing the amount of (co)medication, the method comprising administering alkaline phosphatase to a subject receiving said (co)medication or being at risk of receiving said (co)medication. The inventors have observed that not only the amount of (co)medication was decreased in subjects receiving AP in comparison with subjects receiving placebo, but also that administration of AP reduces adverse effects of (co)medication. Therefore, a method for decreasing adverse effects of (co)medication in a subject receiving said (co)medication or being at risk of receiving said (co)medication is disclosed, the method comprising
administering alkaline phosphatase before, during and/or after said subject receives said medication, thereby decreasing adverse effects of said (co)medication in said subject.
Preferably, a method is disclosed, wherein AP is used for reducing (co)medication for the treatment of a heart condition, preferably a heart condition resulting from myocardial infarction or heart failure.
Preferably, a method is disclosed, wherein the (co)medication is a class III anti-arrhythmic, a selective β-blocker, a digitalis glycoside, a vitamin K antagonist, or a selective β-2-adrenoceptor agonist. The disclosure provides the insight that the need for especially these drugs is lower in subjects receiving AP.
Preferably, a method is disclosed, wherein the (co)medication is potassium, a benzo-derivative, a plain sulfonamide, magnesium and/or digitalis glycosides. As the before mentioned drugs are potentially kidney damaging in subjects exposed to them, reducing the amount of these drugs results in a reduced risk of reduced renal function in these subjects.

As especially (co)medication is decreased that is generally given for the treatment of a heart condition, especially for the treatment of atrial fibrillation, the invention provides the insight that AP is able to prevent and/or treat atrial fibrillation. Therefore, a method for preventing and/or treating atrial fibrillation is disclosed, the method comprising
administering alkaline phosphatase to a subject suffering or at risk of suffering from atrial fibrillation, thereby preventing or treating said atrial fibrillation. In intensive care unit (ICU) patients, the incidence of and morbidity due to atrial fibrillation is relatively high (up to 70%, depending on the kind of ICU).

### Alkaline Phosphatase

Alkaline phosphatase (AP; EC 3.1.3.1 according to IUBMB Enzyme Nomenclature), is an enzyme that catalyzes the reaction of a phosphatase monoester and H₂O to an alcohol and phosphate. Other name(s) for AP are alkaline phosphomonoesterase; phosphomonoesterase; glycerophosphatase; alkaline phosphohydrolase; alkaline phenyl phosphatase; orthophosphoric-monoester phosphohydrolase (alkaline optimum). The systemic name of AP is phosphate-monoester phosphohydrolase (alkaline optimum).

AP is a wide specificity enzyme, it also catalyses transphosphorylations. In humans and other mammals at least four distinct but related AP are known. They are intestinal, placental, placental-like, and liver/bone/kidney (or tissue non-specific) AP. The first three are located together on chromosome 2 while the tissue non-specific form is located on chromosome 1. The exact physiological functions of the APs are not known, but AP appears to be involved in a large number of physiological processes.

A source of AP for use in the invention can be a commercial AP enzyme, or any composition comprising the AP enzyme and any means capable of producing a functional AP enzyme in the context of the current invention, such as DNA or RNA nucleic acids encoding an AP protein. The nucleic acid encoding AP may be embedded in suitable vectors such as plasmids, phagemids, phages, (retro)viruses, transposons, gene therapy vectors and other vectors capable of inducing or conferring production of AP. Also native or recombinant micro-organisms, such as bacteria, fungi, protozoa and yeast may be applied as a source of AP in the context of the current invention.

AP containing compositions for use according to the current invention preferably comprise a eukaryotic AP, more preferably a mammalian AP, which may be of the types tissue non-specific AP, such as liver-bone or kidney type, or tissue specific such as placental AP, intestinal AP and placental-like AP. The latter, also known as germ cell AP, is localized to testis, thymus and certain germ cell tumors (1), and is closely related to both the placental and intestinal forms of AP (2). The skilled person is very well capable of searching nucleic acid libraries and selecting a sequence that encodes AP. Most preferably the mammalian AP is a human or a bovine AP. Hence, preferably, the use of AP in the manufacture of a medicament for improving reduced renal function is disclosed herein, wherein said AP is mammalian AP and even more preferably wherein said AP is human AP. Non-limiting examples of a human AP sequence can be found in the NCBI (Genpept) collection and include: NP_001622 (intestinal AP), NP_001623 (placental AP), NP_112603 (placental-like AP) or NP_000469 (tissue non-specific AP). The invention also comprises the use of a polymorphism of any of said sequence. In yet another preferred embodiment, said AP is placental AP, placental-like AP, intestinal AP or liver/bone/kidney AP.

In yet another preferred embodiment, methods and uses according to the invention are provided wherein the AP is recombinant AP.

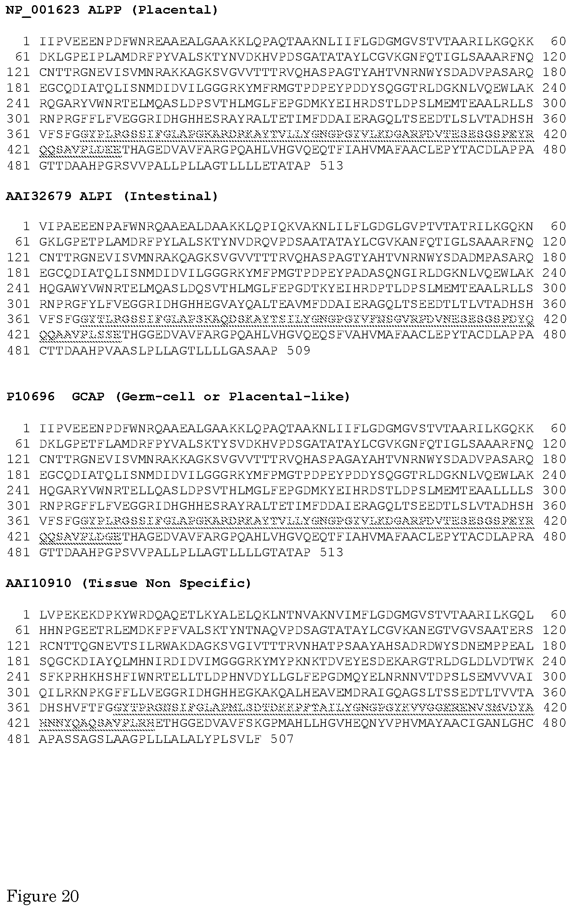

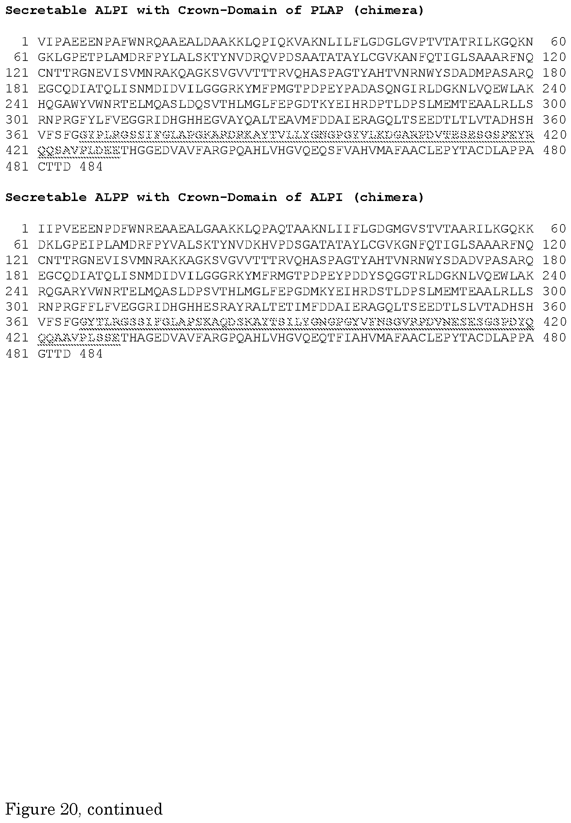

From a conformational point of view, an AP roughly consists of two domains: a crown domain and an active-site domain. The active-site domain can be divided in separate parts like the catalytic residue and the three metal ion sites (Zn1, Zn2 and Mg3). From a primary structure point of view it is clear that the crown domain is flanked by the amino acids that form the active site domain. The amino acid sequence of APs and the relative positions of the catalytic and crown domain are known by the skilled person. As an example, reference is made to Figure 20 which shows, amongst others, the amino acid sequence of the four human APs. The crown domain is underlined in these sequences.

APs are present in virtually all organisms from bacteria to humans. It is preferred that an AP for use in the invention is an isolated or recombinant AP comprising a crown domain and a catalytic domain, wherein said crown domain and said catalytic domain are obtained from different APs and wherein at least one of said different phosphatases is a human phosphatase. The other phosphatase is for example ECAP (*Escherichia coli* AP) or one of the seven known BIAPs (Bovine Intestinal AP). Preferably, an isolated or recombinant AP is used comprising a crown domain and a catalytic domain, wherein said crown domain and said catalytic domain are obtained from different APs and wherein the different APs are human APs. This is especially useful if the modified phosphatase is subsequently used in human therapy. It is expected that such (genetically) modified APs of human origin are not or very little immunogenic. However it is clear to the skilled person that if a modified AP is for example used in "in vitro" or "ex vivo" diagnostics a modified phosphatase may well be composed of for example a human and an *E.coli* AP or may be composed of a bovine and an *E.coli* AP.

An AP for use in the invention is preferably an isolated or recombinant AP comprising a crown domain and a catalytic domain, wherein said crown domain and said catalytic domain are obtained from different APs and wherein said crown domain is the crown domain of placental AP (ALPP) and wherein said catalytic domain is the catalytic domain of intestinal AP (ALPI). Preferably, at least one of said different phosphatases is a human phosphatase and in an even more preferred embodiment, both different phosphatases are human phosphatases.
Other preferred domain swapped mutants suitable for uses according to the present invention and are based on the human APs are listed in Table 1.

**Table 1 domains swapped alkaline phosphatase enzymes**

| **Catalytic domain** | **Crown domain** | **Referred to as** |
|---|---|---|
| ALPI | GCAP | catALPI/crownGCAP |
| | TNAP | catALPI/crownTNAP |
| ALPP | GCAP | catALPP/crownGCAP |
| | TNAP | catALPP/crownTNAP |
| GCAP | ALPI | catGCAP/crownALPI |
| | ALPP | catGCAP/crownALPP |
| | TNAP | catGCAP/crownTNAP |
| TNAP | ALPI | catTNAP/crownALPI |
| | ALPP | catTNAP/crownALPP |
| | GCAP | catTNAP/crownGCAP |

For the sake of clarity, ALPI is intestinal AP, ALPP is placental AP, GCAP is placental-like AP and TNAP is tissue non-specific AP.

It is clear that also combinations between the catalytic domain of ECAP or any of the human forms (ALPI, ALPP, GCAP or TNAP) with the crown domain of BIAP can be made. Moreover, combinations of the crown domain of BIAP with the catalytic domain of ECAP or any of the human forms can also be produced.

Another class of useful modified phosphatases are phosphatases which under natural conditions are linked to the membrane of a cell via a glycosylphosphatidylinositol (GPI) anchor but which are now modified such that they are no longer attached to the membrane of a cell. Examples of phosphatases that are GPI-anchored are AP and 5'-nucleotidase. All isoenzymes are functionally active in the cell membrane and GPI-anchor deficient forms are not naturally present at detectable levels. Although serum alkaline phosphate activity has been demonstrated it is generally accepted that the enzyme is still present in shed membrane fractions or membrane vesicles. AP activity in milk is also present in fractions containing membrane vesicles. The GPI anchor is stored as a precursor molecule in the cell where it is attached to the attachment site through a transamidase. The backbone of the GPI-anchor is identical in mammals, but cell-type dependent modifications are known.

For pharmaceutical use of AP in human subjects it is for most applications preferred to apply human forms of the enzyme for use in medicaments and treatment, as AP forms obtained from other species may be immunogenic in human subjects and treatment could elicit immunological reactions and pathological side effects. In some subjects even lethal side effects i.e. anaphylactic shock may occur and the risks of immunological side effects are therefore preferably minimized by use of human AP forms. As isolation of AP from humans is practically not feasible, human recombinant forms of the AP proteins can be routinely produced in different recombinant expression platforms. However, expression and purification of GPI modified and membrane-anchored proteins is notoriously difficult; GPI proteins are difficult to separate from membranes and difficult to isolate and purify. It is therefore preferred to use an isolated or recombinant AP comprising a modification in the glycosylphosphatidylinositol (GPI) signal sequence, wherein said modification results in a secreted phosphatase, i.e. the phosphatase is not attached to the cell membrane.

Preferably said isolated or recombinant phosphatase comprises a modification in the glycosylphosphatidylinositol (GPI) signal sequence, wherein said modification results in a secreted phosphatase that is biological active, i.e. it shows activity towards a biological (relevant) substrate, is used.

There is no general sequence responsible for the attachment of a GPI anchor, but there is a clear consensus:
1) hydrophobic stretch of amino acids at the C-terminus (at least 11 amino acids, but preferably more than 11 amino acids)
2) Upstream of the hydrophobic region, a spacer of hydrophylic amino acids (5-12 amino acids)
3) GPI is attached to a small amino acid: glycine, aspartic acid, asparagine, alanine, serine or cysteine.
4) The 2 subsequent amino acids downstream of the GPI attachment site must be small amino acids and in the majority of cases they are selected from glycine, aspartic acid, asparagine, alanine, serine or cysteine.

Based on this consensus, the skilled person is capable of mutating this consensus, for example by inserting one or multiple amino acids and disrupting part of the consensus. However in a preferred embodiment, an isolated or recombinant phosphatase comprising a modification in the glycosylphosphatidylinositol (GPI) signal sequence, wherein said modification results in a secreted phosphatase and wherein said modification comprises a mutation or a deletion of the amino acid sequence encompassing the consensus GPI signal sequence is used.

For applications in human therapy it is desired that the resultant modified phosphatase is not or very little immunogenic, i.e. that the modified phosphatase is essentially of human origin. In a preferred embodiment, a use according to the invention is provided, wherein AP is an isolated or recombinant phosphatase comprising a modification in the glycosylphosphatidylinositol (GPI) signal sequence, wherein said modification results in a secreted phosphatase (preferably with activity against a biological relevant substrate) and wherein said phosphatase is a human phosphatase.

Examples of phosphatases that are GPI-anchored are AP and 5'-nucleotidase and hence in a preferred embodiment, an isolated or recombinant phosphatase is used, comprising a modification in the glycosylphosphatidylinositol (GPI) signal sequence, wherein said modification results in a secreted phosphatase and wherein said phosphatase is an AP, for example a human AP, such as for instance human liver-kidney-bone phosphatase, human intestinal AP, or human placental-like alkaline phosphatise.

It is clear that any of the described secretable modified phosphatase can for example be produced by introducing into a host cell a nucleic acid capable of encoding said secretable phosphatase, preferably in operable linkage with regulatory sequences, and allowing said host cell to express said secretable phosphatase and optionally isolating the produced phosphatase from the medium in which the host cell are grown and/or maintained. However, apart from mutations in the above mentioned GPI-attachment sequence, other methods exist that make GPI-anchorless, secreted proteins:
1) After expression as membrane anchored proteins, phospholipases may be used to cleave off the GPI anchor.
2) Interference with the production of the GPI anchor or the use of a cell (type) that is deficient in GPI anchor production may also be used to make a secretable form of an otherwise GPI-anchored protein. Examples of cell lines that have been made to be deficient in GPI anchoring biochemistry are e.g. Jurkat, AM-B, C84, BW, S49, CHO and Raji.
3) Interference with or the use of a cell deficient in transamidases may be used to inhibit attachment of a GPI anchor to the protein, rendering the protein anchorless and secretable. Such a deficient cell has been obtained through mutagenesis in CHO.

It is clear to the skilled person that a modified phosphatase which comprises a crown domain and a catalytic domain, wherein said crown domain and said catalytic domain are obtained from different APs can be further modified and made secretable. Hence, in a preferred embodiment, an AP is used that is an isolated or recombinant phosphatase comprising a modification in the glycosylphosphatidylinositol (GPI) signal sequence, wherein said modification results in a secreted phosphatase and wherein said recombinant phosphatase further comprises a crown domain and a catalytic domain that are obtained from different phosphatases. Non-limiting examples of such (alkaline) phosphatase mutants are provided in Figure 20. Such a combined or "double" mutant results for example in a modified phosphatase with a certain specific activity, stability or substrate specificity and at the same time production of such a product is greatly enhanced by the fact that it can be isolated from the medium surrounding the producer cells. Such recombinant chimeric or mutant alkaline phosphatase has extensively been described in published international patent application WO08/133511.

The AP may be administered via different routes, for example intravenously, rectally, bronchially or orally.

In a preferred embodiment, the used route of administration is intravenously. It is clear for the skilled person, that preferably an effective amount of AP is delivered. As a starting point 10-500 U/kg/day can be used. If the intravenous route of administration is used, AP (at least for a certain amount of time) is preferably applied via continuous infusion.

The invention will be explained in more detail in the following, non-limiting examples.

### DESCRIPTION OF FIGURES

Figure 1: Renal Replacement Therapy (dialysis) Requirement (ITT)
Figure 2: Renal Replacement Therapy Duration (ITT; means/SEM); p: t-test
Figure 3: Mean Relative Duration of Renal Replacement Therapy (hours; ITT; means/SEM); p: t-test; [(cumulative RRT duration)/(time (d) in the study)/*100%
Figure 4: Regression Analysis of Creatinine Clearance 0-7 Days; (AP vs. placebo; FAS; means/SEM)
Figure 5: Progression in Creatinine Clearance (FAS; means/SEM)
Figure 6: Progression in Creatinine Clearance Adjusted for Missing Values (FAS; means/SEM)
Figure 7: Length of Stay in ICU (Subgroup A; ITT; means/SEM)
Figure 8: Requirement for Mechanical Ventilation (ITT; means/SEM)
Figure 9: Changes in SOFA Score (ITT; means/SEM)
Figure 10: Analysis of the biomarker KIM-1 in patients during the first 48 hours of treatment. The graph represents the mean concentration (in ng/ml) in urine of patients (A) and the mean concentration (in ng/mg creat) in urine of patients that required RRT (B). Diamonds denote AP treatment group, triangles denote Placebo group.
Figure 11: Analysis of the biomarker IL-18 in patients during the first 48 hours of treatment. The graph represents the mean concentration (in pg/ml) in urine of patients (A) and the mean concentration (in pg/ml) in urine of patients that required RRT (B). Diamonds denote AP treatment group, triangles denote Placebo group.
Figure 12: Analysis of the biomarker KIM-1 in patients during the first 168 hours after start of treatment. The graph represents the mean concentration (in ng/ml) in urine of patients (A) and the mean concentration (in ng/ml) in patients that required RRT (B). Diamonds denote AP treatment group, triangles denote Placebo group.
Figure 13: Analysis of the biomarker IL-18 in patients during the first 168 hours after start of treatment. The graph represents the mean concentration (in pg/ml) in urine of patients (A) and the mean concentration (in pg/ml) in patients that required RRT (B). Diamonds denote AP treatment group, triangles denote Placebo group.
Figure 14: Analysis of the biomarker CRP in patients during the first 168 hours after start of treatment. The graph represents the mean concentration (in mg/dl) in blood of patients (A) and the mean concentration (in mg/dl) in patients that required RRT (B). Diamonds denote AP treatment group, triangles denote Placebo group.
Figure 15: Analysis of the biomarker LBP in patients during the first 168 hours after start of treatment. The graph represents the mean concentration in blood of patients. Diamonds denote AP treatment group, triangles denote Placebo group
Figure 16: Analysis of the biomarker NGAL in patients during the first 144 hours after start of treatment. The graph represents the mean, creatinine normalized concentration in urine of patients. Diamonds denote AP treatment group, triangles denote Placebo group
Figure 17: Analysis of the biomarker IL-6 in patients during the first 168 hours after start of treatment. The graph represents the mean concentration in blood of patients. Diamonds denote AP treatment group, triangles denote Placebo group

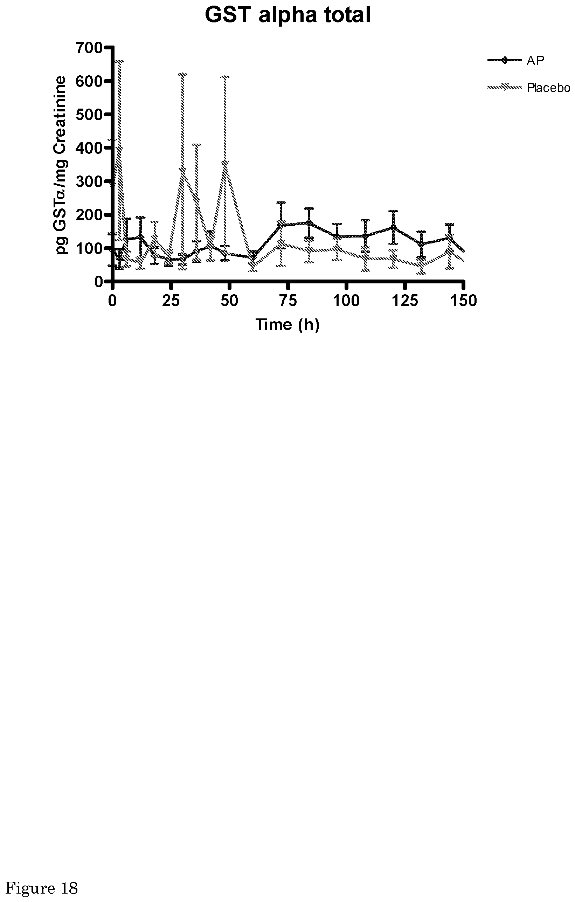

Figure 18: Analysis of the biomarker GST alpha in patients during the first 144 hours after start of treatment. The graph represents the mean, creatinine normalized concentration in urine of patients. Diamonds denote AP treatment group, triangles denote Placebo group

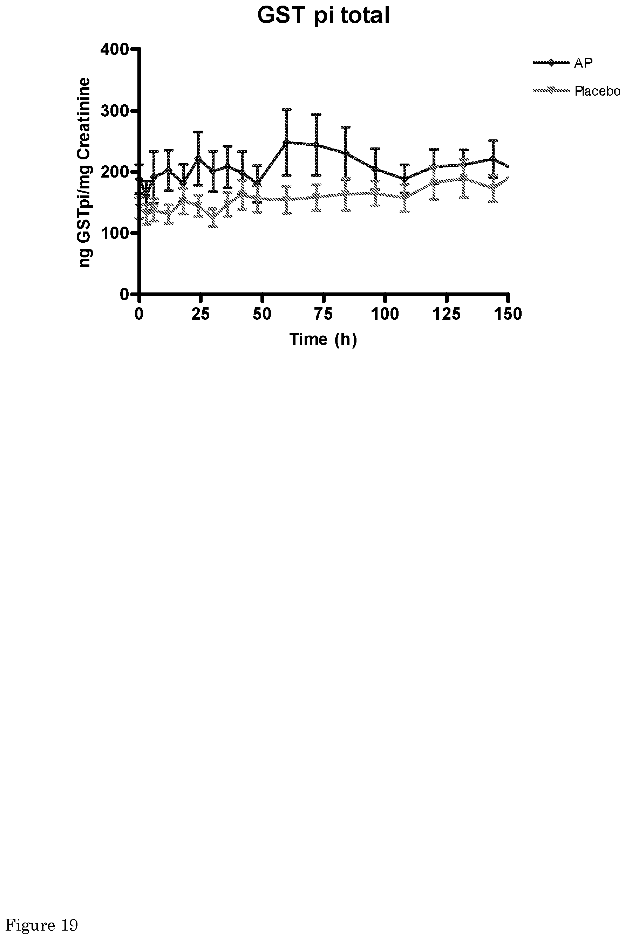

Figure 19: Analysis of the biomarker GST pi in patients during the first 144 hours after start of treatment. The graph represents the mean, creatinine normalized concentration in urine of patients. Diamonds denote AP treatment group, triangles denote Placebo group
Figure 20: Sequences of human alkaline phosphatase enzymes. Depicted are the sequences of the mature proteins (i.e. without signal sequence) but before addition of the GPI-anchor and concomitant processing of the C-terminal amino acids with exception of the chimeric AP's.

### Examples

### Materials and Methods

### Example 1 - Effect of AP on Renal Function in Sepsis Patients

### Patients

A total of 36 patients were randomised and enrolled at six centres in Belgium and The Netherlands. Recruitment across centres ranged from 1-13 patients. Demographics and disease severity parameters at baseline did not suggest any relevant or significant differences between treatment groups, as summarised in Table 2.

**Table 2: Baseline Comparability Between Groups**

| **BASELINE PARAMETER** | **AP (n=16)** | **Placebo(n=19)** |
|---|---|---|
| **Male: n (%)** | 13 (81) | 14 (74) |
| **Age: mean years (SD)** | 65 (12) | 67 (15) |
| **Height: mean cm (SD)** | 176 (10) | 174 (8) |
| **Weight: mean kg (SD)** | 86 (12) | 80 (14) |
| **Heart rate: mean bpm (SD)** | 103 (23) | 105 (22) |
| **Systolic BP: mean mmHg (SD)** | 103 (26) | 110 (26) |
| **Diastolic BP: mean mmHg (SD)** | 52 (13) | 55 (13) |
| **Temperature: mean °C (SD)** | 37 (1) | 37 (1) |
| **APACHE-II score: mean (SD)** | 24 (7) | 23 (8) |
| **SOFA score: mean (SD)** | 10 (4) | 11(5) |
| **Serum creatinine: median µmol/L (IQR)** | 172 (126;198) | 173 (115;268) |
| **Creatinine clearance: mean mL/min (SD)*** | 50 (27) | 40 (37) |

| | | |
|---|---|---|
| Notes: all patients were Caucasians. *: missing values estimated by Cockroft-Gault formula. | | |

### Intervention

Study medication was to be prepared within 1 hour before dosing. The study medication was labelled (as per current regulations) by the Hospital Pharmacy.

The active ingredient was bovine intestinal alkaline phosphatase (BIAP), which is an enzyme of bovine origin, composed of 533 amino acids with a molecular weight of 140kD, and that carries out esterase reactions on phosphate-containing substrates at a high pH optimum under *in vitro* biochemical assay conditions. AP was supplied as a sterile solution for infusion containing AP activity of 13.600U/mL (as determined using glycin buffer at 25°C) in an aqueous buffer containing 5mM Tris-HCl, 5mM Magnesium chloride, 0.1mM zinc chloride, pH 7.2-7.4, with 30-35% (w/v) glycerol as stabiliser. Matching placebo for use in controlled trials consists of a sterile solution for infusion containing an aqueous buffer containing 5mM Tris-HCl, 5mM Magnesium chloride, 0.1mM zinc chloride, pH 7.2-7.4, 30-35% (w/v) Glycerol and Water for Injection (qs).

Patients received an intravenous injection of AP 67.5U/kg over 10 minutes followed by continuous infusion of 132.5U/kg/24h over 48 hours, or placebo, and were followed-up for 28 days.

AP was supplied as a clear to slightly opalescent, colourless, sterile, pyrogen-free solution in 10mL type I glass vials with a Teflon-coated bromobutyl rubber stopper. Each vial contained 1mL of AP solution (13,600 Units).

Matching placebo was supplied as a clear, colourless, sterile, pyrogen-free solution in type I glass vials with a Teflon-coated bromobutyl rubber stopper. Each vial of 1mL placebo contained 5mM Tris-HCl, 5mM Magnesium chloride, 0.1mM zinc chloride, pH 7.2-7.4, with 30-35% (w/v) glycerol as stabilizer and Water for Injection (qs).

All study medication was stored in the Hospital Pharmacy in a lockable storage facility under appropriate pharmacy conditions.

### Requirement for Renal Replacement Therapy

RRT (dialysis) is currently the main intervention for acute kidney injury (AKI) in the ICU setting, while AKI requiring RRT is associated with the worst prognosis. Thus, reduced need for RRT was defined as a primary efficacy endpoint for treatment of AKI in the sepsis setting.

Eligibility (requirement) for RRT was strictly defined in the Protocol for several reasons. Firstly, only recently a consensus on criteria for RRT initiation has emerged, the approach still differing across hospitals and between physicians in the same hospital. Therefore, the EMEA advised the adoption of strict definition for RRT requirement, in line with the Acute Dialysis Quality Initiative International (ADQII) Consensus, which consists of the following criteria (incorporated in the Protocol):
1. Oliguria: < 0.3mL/Kg/h for >6h AND serum urea >20mmol/L, OR
2. Anuria: >12h AND serum urea >15mmol/L, OR
3. Hyperkalaemia: >7.5mmol/L or with typical ECG changes despite therapeutic intervention, OR
4. Acidosis with standard bicarbonates <15mmol/L despite therapeutic intervention, OR
5. Hyponatraemia: <120mmol/L despite therapeutic intervention, OR
6. Clinical diagnosis of fluid overload with established or threatening pulmonary or brain oedema despite therapeutic intervention.

In addition, the Dutch authorities (CCMO, Central IRB) mandated that patients already on RRT at entry must be excluded from the Protocol.

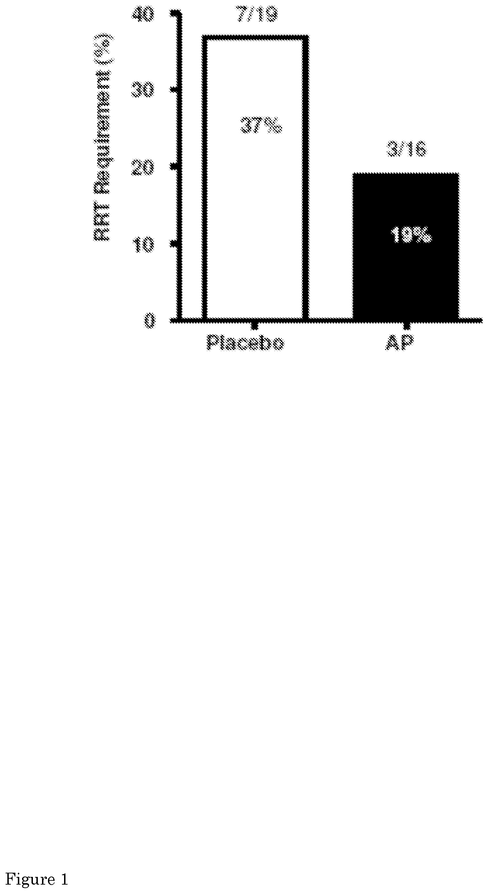

The results for RRT requirement (Yes-No analysis) are illustrated in Figure 1, with numerical data displayed in Table 3.

**Table 3: Renal Replacement Therapy Requirement and Duration (ITT)**

| | **AP (n=16)** | **Placebo (n=19)** |
|---|---|---|
| **RRT required: n** | 3 (19%) | 7 (37%) |
| **RRT not required: n** | 13 (81%) | 12 (63%) |
| **p*** | 0.2853 | |
| **Mean duration of RRT: hours (SD)** | 10 (23) | 53 (99) |
| **p**** | 0.0801 | |
| **Relative RRT Duration (patients requiring RRT): % (SD)** | 12 (4.8) | 34 (15.8) |
| **p**** | 0.0456 | |

| | | |
|---|---|---|
| *: Fisher's exact test; **: T-test [(Cumulative RRT duration)/(Time (d) in the study)]*100% | | |

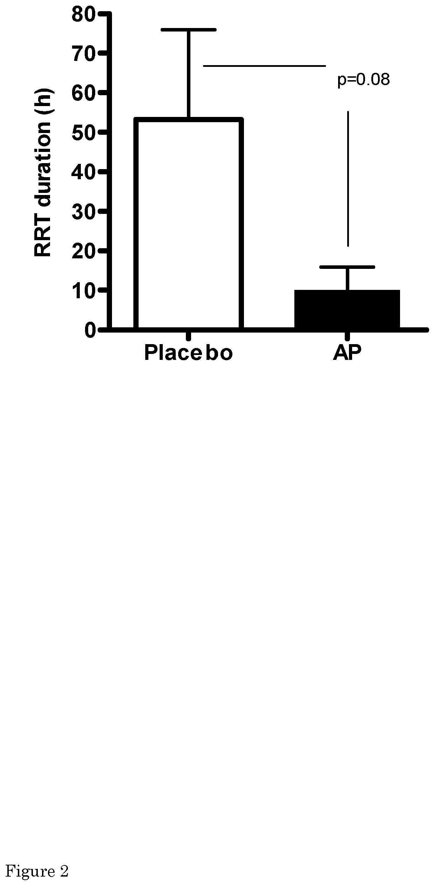

The results in Figure 1 and Table 3 demonstrate that the placebo group had approximately a 2-fold greater requirement for RRT interventions (37%) than the AP group (19%). Mean duration of RRT showed a strong trend (p=0.0801; t-test) in favour of the AP-treated group with 10±23h, relative to 53±99h on placebo, as displayed in Figure 2.

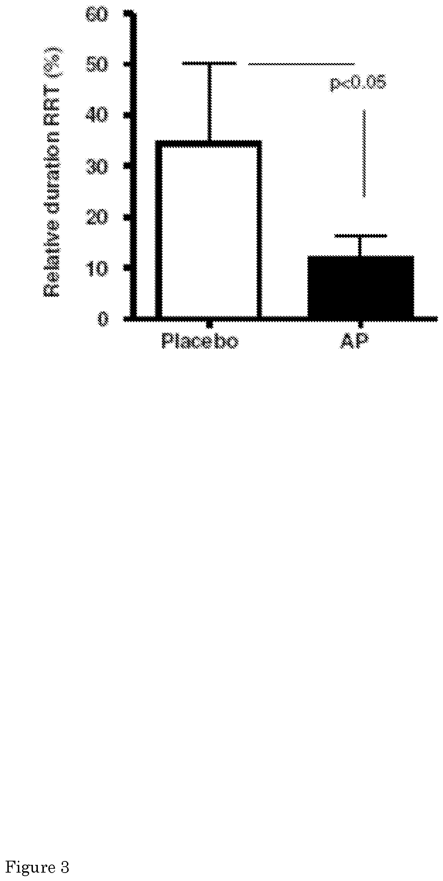

Relative duration of RRT for patients requiring dialysis was also significantly lower in the AP group (p=0.0456), compared to placebo (Figure 3).

### Creatinine Clearance

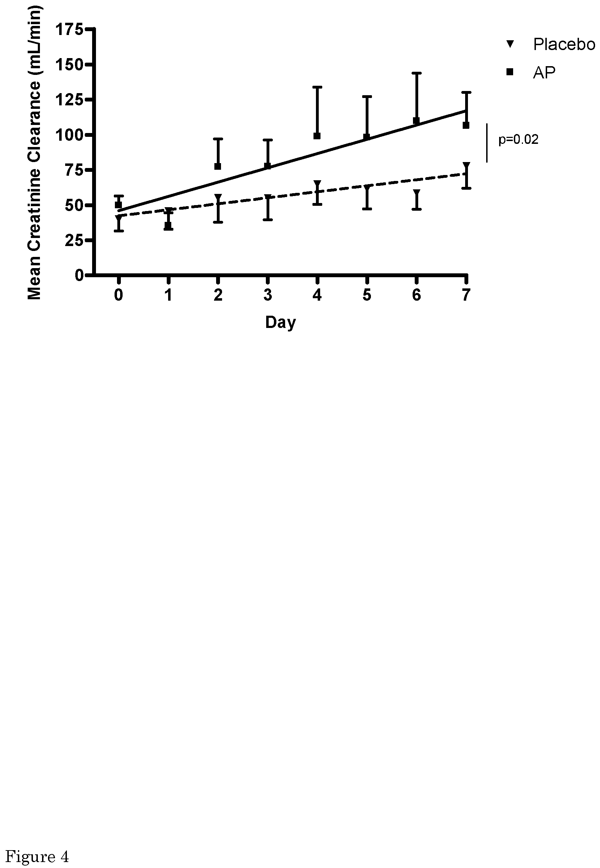

Creatinine clearance is arguably the most appropriate measurement of kidney function. Importantly, unlike serum creatinine levels, creatinine clearance is to a much lesser extent influenced by RRT, thus being a more robust indicator of renal function in this setting. AUCs of creatinine clearance were calculated using creatinine levels in serum and urine, urinary output and body surface area. Where 24h urinary output was not available (at baseline), the Cockroft-Gault formula was used to estimate clearance. Missing values were imputed from last last-observation-carried forward (LOCF) or linear interpolation.
To enable estimation of treatment effect on creatinine clearance over time, regression analysis was conducted to elucidate a possible relationship of either treatment-group with direction of effect and time. The regression analysis of values corrected for baseline confirmed a highly significant (p=0.0181) and progressive effect of AP on clearance levels up to Day 7, relative to placebo, as shown in Figure 4.

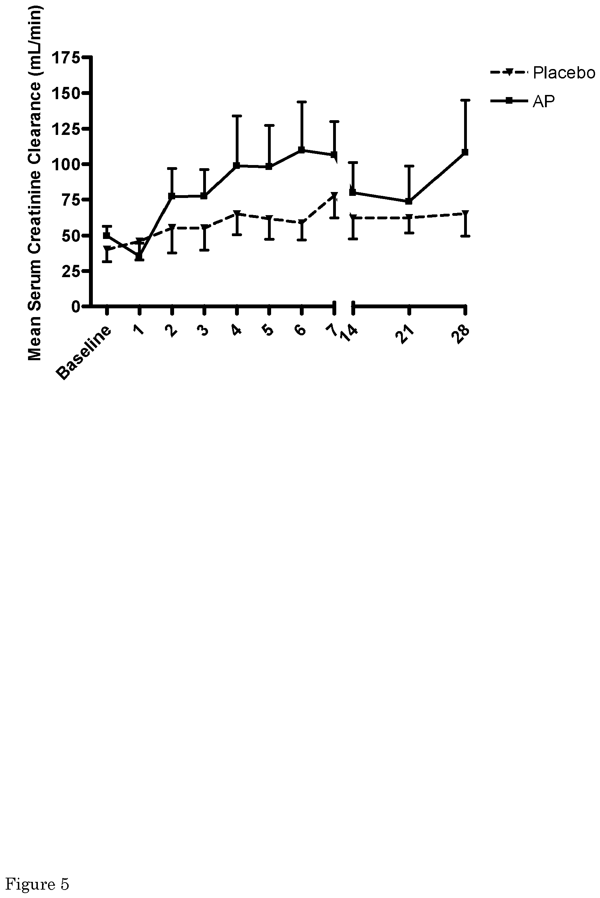

Figure 5 displays the progress of creatinine clearance per time point throughout the 28 days' study period. Thus, the observed treatment effect suggests an improvement in creatinine clearance in the treated group to normal levels (~100mL/min), relative to placebo where clearance remains impaired.

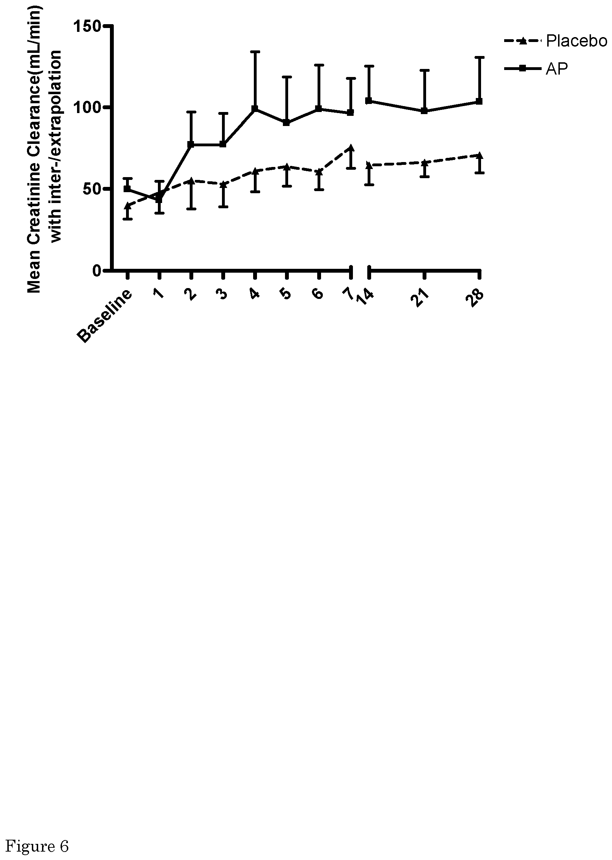

In Figure 5, after Day 14 the data are influenced by there being very few patients still in ICU, particularly in the AP group (n=4), because outside ICU 24-hour urinary output is not routinely collected. Consequently, the means in Figure 5 are an underestimation of the full patient sample due to the absence of patients who improved and left ICU. An estimation of clearance is presented in Figure 6 with missing values imputed from last last-observation-carried forward (LOCF) or linear interpolation. Creatinine clearance over the 28 day period was significantly superior for the AP-treated group relative to placebo (p=0.0199; repeated measures ANOVA).

Thus, the results of creatinine clearance data confirm that the treatment effect is superior for the AP group, both during the first 7 days and throughout the 28-day study duration.

### Serum Creatinine

Serum creatinine is widely used for evaluation of renal function in clinical practice. However, serum creatinine is not optimal because it is a late marker of tissue damage. Serum creatinine only starts to increase after considerable damage has already occurred to nephrons; hence relatively small increases in levels have major influence on outcomes. In addition, serum creatinine levels are greatly affected by RRT intervention. Therefore, serum creatinine levels are pointers of direction of results but do not reflect renal function in the presence of RRT.

According to the Protocol, serum creatinine samples were taken at study Days 1, 2, 3, 4, 5, 6, 7, 14, 21 and 28. However, as in ICUs creatinine is determined daily, all serum creatinine values that were available were collected and entered in the database in order to get as complete as possible a representation over the entire study period.

Owing to the larger proportion of patients on RRT in the placebo group (n=7) relative to the AP group (n=3) and the longer duration of RRT in placebo patients, the actual magnitude of the RRT effect cannot be reliably estimated, but it can be safely assumed that the observed creatinine levels on placebo are an underestimation, as the actual values would be considerably higher without RRT intervention.

AUCs of serum creatinine values were calculated using serum creatinine levels as per biochemistry results. Missing values were imputed from last last-observation-carried forward (LOCF) or linear interpolation. The differences in AUCs of serum creatinine for the periods 0-7 and 0-28 days, as displayed in Table 4, did not reach significance.

**Table 4: AUC Serum Creatinine (µmol/L; FAS)**

| | **AP** | **Placebo** | **Q1; Q3** | |
|---|---|---|---|---|
| | | | **AP** | **Placebo** |
| **Mean (SD) 0-7 days** | 1099 (510) | 1411 (843) | 725; 1265 | 710; 1977 |
| **Mean (SD) 0-28 days** | 4091(2623) | 4284 (2920) | 2298; 4833 | 2400; 4562 |

| | | | | |
|---|---|---|---|---|
| Lower AUC values denote better renal function. | | | | |

The above results confirm that for the 28-day period serum creatinine levels in the AP group were similar to, albeit slightly better than on placebo. The observed difference is an underestimation due to the substantial creatinine lowering effect of intense RRT required by patients on placebo.

### Combined Treatment Effects on Efficacy Parameters

A combined analysis of all main efficacy variables was conducted as per Statistical Plan, to evaluate global treatment effect on renal function by an established accepted method when all main parameters are considered. The results are displayed in Table 5.

**Table 5: Analysis of Combined Significances of Variables (ITT)**

| Legend: | | |
|---|---|---|
| A: RRT requirement (0-28d) | | |
| B: RRT cumulative duration (0-28d) | | |
| C: RRT relative duration (0-28d) | | |
| D: Creatinine Clearance linear regression (0-7d) | | |
| E: AUC Serum creatinine without RRT correction (0-7d; day 0 as covariate) | | |
| **Combined Parameters** | **p-value*** | |
| A+C+D+E | 0.005 | |
| B+D+E | 0.001 | |
| A+C+D | 0.018 | |
| B+D | 0.016 | |

| | | |
|---|---|---|
| *: one-sided | | |

### Secondary Efficacy Parameters

The following key severity-related parameters were examined and are summarised: SOFA Score, length of hospital/ICU stay, length of mechanical ventilation and mortality. As shown before, the two treatment groups were balanced at entry (see Table 2). Therefore, the following sections display differences at relevant points during the study, relative to baseline.

### Length of Stay in Intensive Care and in Hospital

Length of ICU stay was considerably shorter for the AP group relative to placebo in all analyses and in all subgroups.

Length of ICU and hospital stay was recorded for the full duration of hospitalisation. Since deaths may bias length of stay, this parameter was analysed without deaths during the study period (subgroup A) and including deaths (subgroup B; where death was considered maximum stay, i.e. 28 days). In both analyses the results showed a clear advantage for the AP group. Mean stay at ICU was significantly shorter by >2-fold for the AP group in subgroup A (p=0.0174), while subgroup B showed a trend in favour of the AP group (p=0.1099). Total length of hospital stay also showed longer stay for placebo by 42% relative to the AP group in subgroup A, although the difference did not reach significance.

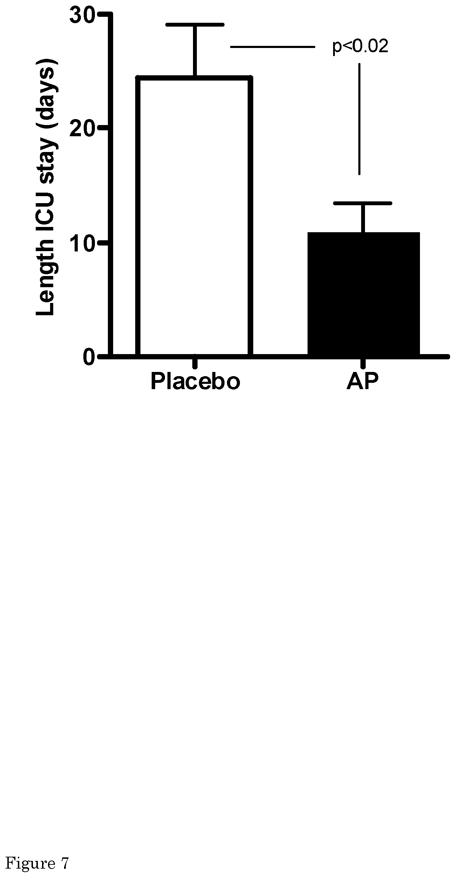

The analysis of length of ICU stay confirmed that shorter overall hospitalisation was not achieved at the expense of longer ICU stay, as displayed in the following Table 6 (subgroups A and B) and in Figure 7 (subgroup A).

**Table 6: Length of Stay in ICU and Hospital (n=35)**

| | **AP** | **Placebo** |
|---|---|---|
| **Subgroup A** | | |
| **ICU Stay (days): mean (SD)** | 10.9 (7.5) | 24.5 (17.9) |
| **AP-Placebo (95% CI)** | 13.6 (-26.6; -0.5) | |
| **p*** | 0.0174 | |
| | | |
| **Hospital Stay (days): mean (SD)** | 33.4 (26.4) | 47.1 (35.7) |
| **AP-Placebo (95% CI)** | -13.7 (-42.2; 14.8) | |
| **p*** | 0.3305 | |
| | | |
| **Subgroup B** | | |
| **ICU Stay (days): mean (SD)** | 13.9 (9.8) | 23.6 (17.6) |
| **AP-Placebo (95% CI)** | -9.7 (-21.8; 2.4) | |
| **p*** | 0.1099 | |

| | | |
|---|---|---|
| *: t-test; A = excluding deaths; B = including deaths (counted as 28 days' stay) | | |

### Length of Mechanical Ventilation

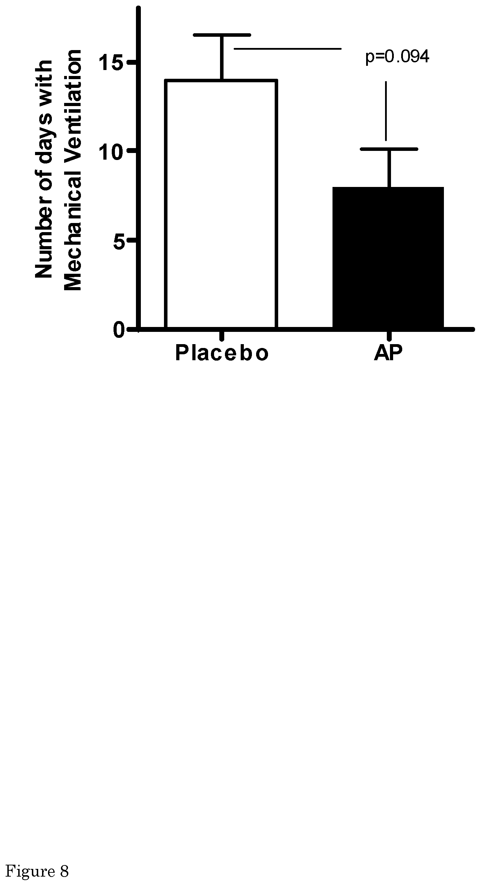

Length of mechanical ventilation was recorded for the full duration of the requirement. Typically, mechanical ventilation takes place in ICU, and 13 patients in both groups were already on mechanical ventilation at baseline. The results showed a strong trend for the AP group relative to placebo (p=0.094). Mechanical Ventilation requirement is directly related to survival. The magnitude of the difference represents a 74% longer requirement of mechanical ventilation on placebo, as displayed in Table 7 and Figure 8.

**Table 7: Length of Required Mechanical Ventilation (ITT)**

| | **AP** | **Placebo** |
|---|---|---|
| **MV Duration (days): mean (SD)** | 8.0 (8.3) | 13.9 (11.3) |
| **AP-Placebo (95% CI)** | -5.9 (-12.8; 1.1) | |
| **p*** | 0.094 | |

| | | |
|---|---|---|
| *: t-test | | |

### SOFA Score

SOFA is a scoring system widely used in clinical trials of sepsis with multiple organ failure. Thus, the SOFA score reflects the overall severity of patients in ICU, related to organ failure by evaluating six organ systems, whereby each contributes a maximum of 4 points; hence the maximum total score is 24 points, higher points indicating worse condition. The SOFA score is directly related to survival. A SOFA score decrease by 2 points is considered clinically meaningful. The mean baseline SOFA score was 10.4 (SD=3.6) and 10.6 (SD=5.3) in the active and placebo groups, respectively, and the score items are displayed in Table 8.

**Table 8: SOFA Scoring System Items**

| **Respiratory System: PaO2**/**FiO2 (mmHg)** | **Score** | **Liver: Bilirubin (mg/dL)** | **Score** |
|---|---|---|---|
| >400 | **0** | <1.2 | **0** |
| <400 | **1** | 1.2-1.9 | **1** |
| <300 | **2** | 2.0-5.9 | **2** |
| <200 and mechanically ventilated | **3** | 6.0-11.9 | **3** |
| <100 and mechanically ventilated | **4** | >12.0 | **4** |

| **Central Nervous System: Glasgow Coma Scale** | | **Coagulation: platelet x10³/µL** | |
|---|---|---|---|
| 15 | **0** | >150 | **0** |
| 13-14 | **1** | <150 | **1** |
| 10-12 | **2** | <100 | **2** |
| 6-9 | **3** | <50 | **3** |
| <6 | **4** | <20 | **4** |

| **Cardiovascular System (mean arterial pressure / administration of vasopressor required)** | | **Renal System: creatinine (mg/dL) (or urine output)** | |
|---|---|---|---|
| No hypotension | **0** | <1.2 | **0** |
| MAP <70mmHg | **1** | 1.2-1.9 | **1** |
| Dopamine ≤5 or Dobutamine (any dose) | **2** | 2.0-3.4 | **2** |
| Dopamine >5 or Epinephrine/Noradrenaline ≤0.1 | **3** | 3.5-4.9 (or <500mL/d) | **3** |
| Dopamine >15 or Epinephrine/Norepinephrine >0.1 | **4** | >5.0 (or <200mL/d) | **4** |

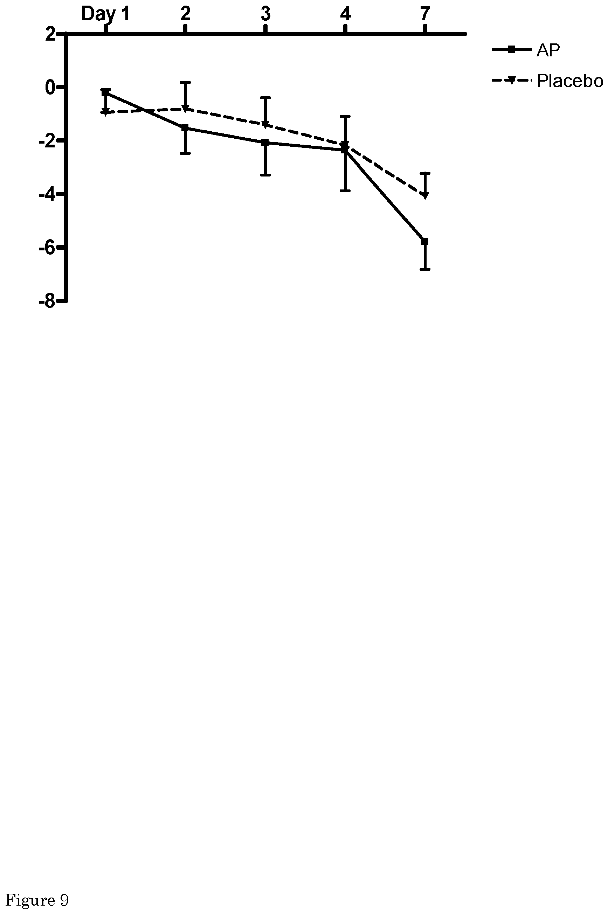

SOFA scores were recorded up to Day 28. Data beyond Day 7 are less reliable as the numbers of patients are small, particularly in the AP group, due to earlier discharge from ICU. Although the sample size was not powered to detect treatment differences in SOFA scores, the AP group had approximately 30% greater improvement than placebo by Day 7, as illustrated in Figure 9.

### Mortality

All-cause mortality at Day 28 was relatively low (n=12; 33%) with no significant differences between treatment groups. Overall, there were seven deaths on AP and five on placebo (Table 9). The causes of deaths were attributed to the underlying condition in all cases.

**Table 9: Mortality Status**

| **SAF Group** | **AP (n=16)** | **Placebo (n=20)** |
|---|---|---|
| **Deaths** | 7 | 5 |
| **p*** | p=0.2532 | |
| | | |

| **PP Group** | **AP (n=14)** | **Placebo (n=18)** |
|---|---|---|
| **Deaths** | 5 | 3 |
| **p*** | p=0.2653 | |

| | | |
|---|---|---|
| * Log-rank | | |

All death reports were reviewed by the independent DSMB and did not raise safety concerns.

### Adverse Events

The incidence and type of adverse events (AEs) were as expected for a population with sepsis and renal failure. At the point of final database lock, the overall event incidence was similar in the two treatment groups (AP: 94%; placebo: 100%). Serious adverse events reflected the severity of disease.

All safety data were reviewed by the independent DSMC who noted that whilst their remit did not strictly include efficacy considerations, nevertheless they observed favourable trends in laboratory tests for AP-treated patients, particularly in terms of renal parameters. The DSMC concluded that AP has a favourable safety profile in the seriously ill patients studied and has a potential for a positive risk:benefit balance in this group.

A summary of reported AEs and SAEs is presented in Table 10 below.

**Table 10: Summary of AEs and SAEs (SAF)**

| | | **AP** | **Placebo** |
|---|---|---|---|
| **ALL AEs** | n (%) | 130 (100) | 154 (100) |
| **Treatment-emergent AEs** | n (%) | 124 (95) | 147 (96) |
| **Patients with Treatment-emergent AEs** | n (%) | 15 (94) | 20 (100) |
| **Non-serious Treatment-** | | | |
| **emergent AEs*:** | | | |
| Atrial fibrillation | | 3 | 6 |
| Diarrhoea | | 6 | 3 |
| Hypotension | | 2 | 7 |
| Delirium | | 2 | 5 |
| Decubitus ulcer | | 1 | 4 |
| Abdominal pain | | 3 | 2 |
| Pyrexia | | 1 | 4 |
| Impaired gastric | | | |
| emptying | | 3 | 2 |
| Tracheostomy | | 1 | 3 |
| Constipation | | 1 | 3 |
| Restlessness | | 2 | 2 |
| Atrial flutter | | 1 | 3 |
| Tachycardia | | . | 3 |
| Hypertension | | 2 | 1 |
| Oedema peripheral | | 2 | 1 |
| Pneumothorax | n | . | 3 |
| Pain | | 1 | 2 |
| Confusional state | | 1 | 2 |
| Hypokalaemia | | 3 | . |
| Agitation | | 1 | 2 |
| Insomnia | | 1 | 2 |
| Haemoglobin decreased | | 2 | 1 |
| Oxygen saturation | | | |
| decreased | | 3 | . |
| Herpes simplex | | 1 | 1 |
| Metabolic acidosis | | 1 | 1 |
| Haematoma | | 1 | 1 |
| Weaning failure | | . | 2 |
| Post procedural | | | |
| haemorrhage | | 1 | 1 |
| Rash | | 1 | 1 |
| Hyperglycaemia | | 1 | 1 |
| Blood phosphorus | | 2 | . |
| decreased | | | |
| Vomiting | | 1 | 1 |
| Back pain | | 2 | . |
| Tachypnoea | | . | 2 |
| **All Serious Treatment-** | | | |
| **emergent AEs** | | | |
| Septic shock | | 2 | 2 |
| Respiratory failure | | 3 | 1 |
| Gastrointestinal necrosis | | 2 | . |
| Hypotension | | . | 2 |
| Death | | 2 | . |
| Hepatic necrosis | | 1 | . |
| Gallbladder necrosis | | 1 | . |
| Electrolyte imbalance | | 1 | . |
| Azotaemia | | 1 | . |
| Cardiac arrest | | . | 1 |
| Bradycardia | | . | 1 |
| Electrocardiogram QT | n | | |
| prolonged | | . | 1 |
| Blood calcium decreased | | . | 1 |
| Coma | | . | 1 |
| Hyperlactacidaemia | | . | 1 |
| Depressed level of | | | |
| consciousness | | . | 1 |
| Osteomyelitis | | 1 | . |
| Brain neoplasm | | . | 1 |
| Renal failure | | . | 1 |
| Therapy cessation | | 1 | . |
| Echocardiogram | | | |
| abnormal | | 1 | . |

| | | | |
|---|---|---|---|
| *: includes >1 event reported in either treatment group Review of the safety data by the independent DSMB did not raise a current safety concern. | | | |

**Concomitant medication**

| CONCOMITANT MEDICATION: CARDIOVASCULAR DRUGS BY ATC-CODE | Treatment group | |
|---|---|---|
| | Placebo | AP |
| | n | n |
| ADRENERGIC AND DOPAMINERGIC AGENTS | 132 | 102 |
| ANTIARRHYTHMICS, CLASS III | 41 | 20 |
| BETA BLOCKING AGENTS, SELECTIVE | 32 | 20 |
| DIGITALIS GLYCOSIDES | 23 | 5 |
| ACE-INHIBITORS, PLAIN | 13 | 9 |
| ANTIARRHYTHMICS, CLASS IB | 9 | 6 |
| VITAMIN K ANTAGONISTS | 9 | 3 |
| DIHYDROPYRIDINE DERIVATIVES | 7 | 4 |
| SELECTIVE BETA-2-ADRENOCEPTOR AGONISTS | 8 | 2 |
| ACTH | 4 | 4 |
| SYMPATHOMIMETICS, PLAIN | 4 | 4 |
| DRUGS FOR TREATMENT OF HYPERKALEMIA | 2 | 3 |
| BETA BLOCKING AGENTS, NON-SELECTIVE | . | 4 |
| VASOPRESSIN AND ANALOGUES | . | 4 |
| ANGIOTENSIN II ANTAGONISTS, PLAIN | 1 | 2 |
| THIAZIDES, PLAIN | 2 | 1 |
| OTHER VASODILATORS USED IN CARDIAC DISEASES | . | 3 |
| ALPHA - ADRENOCEPTOR BLOCKING AGENTS | . | 2 |
| ANGIOTENSIN II ANTAGONIST CALCIUM CHANNEL BLOCK | . | 2 |
| ALPHA AND BETA BLOCKING AGENTS | . | 2 |
| OTHER PERIPHERAL VASODILATORS | . | 1 |
| ANTIARRHYTHMICS, CLASS IC | . | 1 |
| ANGIOTENSIN II ANTAGONISTS AND DIURETICS | . | 1 |
| OTHER CARDIAC PREPARATIONS | 1 | . |
| All | 288 | 205 |

| CONCOMITANT MEDICATION: CV DRUGS WITH VASOPRESSING OR VASODILATING ACTIONS BY ATC-CODE | Treatment Group | |
|---|---|---|
| | Placebo | AP |
| | n | n |
| ADRENERGIC AND DOPAMINERGIC AGENTS | 132 | 102 |
| BETA BLOCKING AGENTS, SELECTIVE | 32 | 20 |
| DIGITALIS GLYCOSIDES | 23 | 5 |
| ACE-INHIBITORS, PLAIN | 13 | 9 |
| VITAMIN K ANTAGONISTS | 9 | 3 |
| ACTH | 4 | 4 |
| SYMPATHOMIMETICS, PLAIN | 4 | 4 |
| DRUGS FOR TREATMENT OF HYPERKALEMIA | 2 | 3 |
| VASOPRESSIN AND ANALOGUES | . | 4 |
| ANGIOTENSIN II ANTAGONISTS, PLAIN | 1 | 2 |
| THIAZIDES, PLAIN | 2 | 1 |
| OTHER VASODILATORS USED IN CARDIAC DISEASES | . | 3 |
| ALPHA - ADRENOCEPTOR BLOCKING AGENTS | . | 2 |
| ANGIOTENSIN II ANTAGONIST CALCIUM CHANNEL BLOCK | . | 2 |
| ALPHA AND BETA BLOCKING AGENTS | . | 2 |
| OTHER PERIPHERAL VASODILATORS | . | 1 |
| ANGIOTENSIN II ANTAGONISTS AND DIURETICS | . | 1 |
| All | 222 | 168 |

### Biomarkers and sampling

To investigate the effect of AP on inflammatory parameters in sepsis patients with renal failure the following biomarkers were analyzed:

### Urine

- Kidney Injury Molecule-1 (KIM-1), increased expression in proximal tubule
- Interleukin-18 (IL-18)
- Lipocalin-2/Neutrophil Gelatinase Associated Lipocalin (NGAL)

Note: Assays for α- or π-Glutathione S-Transferase (α- GST or π-GST) await validation and results of the analysis will be available soon.

### Blood

- Lipopolysaccharide Binding Protein (LBP)
- C-reactive Protein (CRP)
- Interleukin-6 (IL-6)

### Sampling

Samples were collected at regular intervals (Tables 11-13) and frozen immediately:

**Table 11. Sampling schedule for urinary biomarkers**

| Sample time points | Time cumulative (h) |
|---|---|
| Day 1, t=0, 3, 6, 12,18 | 0, 3, 6, 12, 18 |
| Day 2, t=0, 6, 12, 18 | 24, 30, 36, 42 |
| Day 3, t=0, 12 | 48, 60 |
| Day 4, t=0, 12 | 72, 84 |
| Day 5, t=0, 12 | 96, 108 |
| Day 6, t=0, 12 | 120, 132 |
| Day 7, t=0, 12 | 144, 156 |
| Day 8, t=0, 12 | 168, 180 |
| Day 14, t=0, 12 | 336, 348 |
| Day 21, t=0, 12 | 504, 516 |
| Day 28, t=0, 12 | 672, 684 |

**Table 12. Sampling schedule for blood biomarkers (LBP, IL-6)**

| Sample time points | Time cumulative (h) |
|---|---|
| Day 1, t=0, 12 | 0, 12 |
| Day 2, t=0, 12 | 24, 36 |
| Day 3, t=0, 12 | 48, 60 |
| Day 4, t=0 | 72 |
| Day 5, t=0 | 96 |
| Day 6, t=0 | 120 |
| Day 7, t=0 | 144 |
| Day 8, t=0 | 168 |

**Table 13. Sampling schedule for blood biomarker (CRP)**

| Sample time points | Time cumulative (h) |
|---|---|
| Day 1, t=0 | 0 |
| Day 2, t=0 | 24 |
| Day 3, t=0 | 48 |
| Day 4, t=0 | 72 |
| Day 5, t=0 | 96 |
| Day 6, t=0 | 120 |
| Day 7, t=0 | 144 |
| Day 8, t=0 | 168 |
| Day 14, t=0 | 336 |
| Day 21, t=0 | 504 |
| Day 28, t=0 | 672 |

### Biomarker analysis

### NGAL

Neutrophil gelatinase-associated lipocalin (NGAL) was determined by means of ELISA. Capture antibody and detection antibody were obtained from R&D systems (Abingdon, UK). Capture antibody was diluted in sodium carbonate buffer pH 9.6 and pipetted in Nunc Maxisorp 96-wells microtiter plates. After incubation during 12 hours at 4°C plates were washed three times with 50 mM PBS, pH 7.4 containing 0.1% Tween-20 (ELx50, BioTek Instruments, Winooski, VT, USA). Urine samples were diluted 10, 100, or 1000 times in 0,1% BSA in PBS. Standard curves were made from purified NGAL, ranging from 20 to 0 ng/ml. Samples and standards were incubated for 1 hour at room temperature (RT). After three times washing, detection antibody, diluted in 1% BSA in PBS, was added and incubated for 1 hour at RT. After washing 100 µl Streptavidin-HRP in 1% BSA in PBS was added and incubated for 20 minutes at RT. The plate was washed again and then 100 µl substrate solution, Citrate-phosphate buffer pH 5 + 0.05% O-phenylenediamine (OPD) + 0.03% H₂O₂ was added and colour formation was stopped after 15 minutes RT by adding 0.5 M H2SO4. The plate was read at 490 nm in a Biotek plate reader (Powerwave 200, (BioTek Instruments, Winooski, VT, USA)

### Kim-1

Kidney injury marker-1 (Kim-1) was determined by means of ELISA. Capture antibody and detection antibody were obtained from R&D systems. Capture antibody was diluted in sodium carbonate buffer pH 9.6 and pipetted in Nunc Maxisorp 96-wells microtiter plates. After incubation during 1 hour at room temperature plates were washed three times with 50 mM PBS, pH 7.4 containing 0.1% Tween-20 (ELx50, BioTek Instruments, Winooski, VT, USA). Urine samples were diluted 2 times in 0,1% BSA in PBS. Standard curves were made from purified KIM-1, ranging from 8 to 0 ng/ml. Samples and standards were incubated for 1 hour at room temperature (RT). After three times washing, detection antibody, diluted in 1% BSA in PBS, was added and incubated for 1 hour at RT. After washing 100 µl Streptavidin-HRP in 1% BSA in PBS was added and incubated for 20 minutes at RT. The plate was washed again and then 100 µl substrate solution, Citrate-phosphate buffer pH 5 + 0.05% O-phenylenediamine (OPD) + 0.03% H₂O₂ was added and colour formation was stopped after 15 minutes RT by adding 0.5 M H2SO4. The plate was read at 490 nm in a Biotek plate reader (Powerwave 200, (BioTek Instruments, Winooski, VT, USA)

### GST-pi

Glutathione S-Transferase (GST)-pi was determined by means of EIA. Polysorp-NUNC plates were coated with purified GST-pi (0,33 µg/ml, Abnova, Taipei City Taiwan) in each well 100 µl sodium carbonate buffer pH 9.6 for 16 hours at 4°C. Undiluted urine samples (60 µl) and anti-human GST-pi antibody (Immundiagnostik, Bensheim, Germany, AK3105) in 1% BSA-PBS (60 µl) were also incubated for 12 hours at 4°C in separate Greiner round bottem plates. Standard curves were made from purified GST-pi, ranging from 82 to 0 ng/ml. After 12 hours the Polysorp plate was washed three times and 100 µl of the sample/antibody mix was pipette into the Polysorp plate. After washing 100 µl Streptavidin-HRP in 1% BSA in PBS was added and incubated for 20 minutes at RT. The plate was washed again and then 100 µl substrate solution, Citrate-phosphate buffer pH 5 + 0.05% O-phenylenediamine (OPD) + 0.03% H₂O₂ was added and colour formation was stopped after 15 minutes RT by adding 0.5 M H2SO4. The plate was read at 490 nm in a Biotek plate reader (Powerwave 200, (BioTek Instruments, Winooski, VT, USA).

### GST alpha

Glutathione S-Transferase (GST)-alpha was determined by means of EIA. Maxisorp-NUNC plates were coated with purified GST-alpha (0,9 µg/ml, Abnova, Taipei City Taiwan) in each well 100 µl sodium carbonate buffer pH 9.6 for 16 hours at 4°C. Undiluted urine samples (60 µl) and anti-human GST-alpha antibody (Abnova) in 1% BSA-PBS (60 µl) were mixed in separate Greiner round bottem plates. After 16 hours the Maxisorp plate was washed three times and 100 µl of the sample/antibody mix was pipetted into the Maxisorp plate. Standard curves were made from purified GST-alpha, ranging from 900 to 0 pg/ml. After incubation of 1 hour at RT and washing 100 µl Rabbit anti Mouse biotin-labeled antibody in 1% BSA in PBS was added and incubated for 20 minutes at RT. The plate was washed again and then 100 µl substrate solution, Citrate-phosphate buffer pH 5 + 0.05% O-phenylenediamine (OPD) + 0.03% H₂O₂ was added and colour formation was stopped after 45 minutes RT by adding 0.5 M H2SO4. The plate was read at 490 nm in a Biotek plate reader (Powerwave 200, (BioTek Instruments, Winooski, VT, USA).

### IL-18

NUNC maxisorp plates were coated with 100 µl IL-18 antigen (Randox, Crumlin, UK) concentration 125 pg/ml in carbonate buffer pH 9.6 and incubated 16 hours at 4°C. Undiluted urine samples (60 µl) was pipetted in a roundbottem Greiner plate and 60 µl binding antibody (Randox, sheep anti-IL-18 polyclonal antibody) was added to the samples and incubated 16 hours at 4°C. Maxisorp plates were washed and 100 µl of the sample/antibody mixture was pipette into the maxisorp plate. Standard curves were made from purified IL-18, ranging from 125 to 0 pg/ml. After three times washing, Rabbit anti Sheep HRP-labeled antibody, diluted in 1% BSA-PBS was added and incubated for 1 hour at RT. The plate was washed again and then 100 µl substrate solution, Citrate-phosphate buffer pH 5 + 0.05% O-phenylenediamine (OPD) + 0.03% H₂O₂ was added and colour formation was stopped after 45 minutes RT by adding 0.5 M H2SO4. The plate was read at 490 nm in a Biotek plate reader (Powerwave 200, (BioTek Instruments, Winooski, VT, USA).

### Statistical analysis

Data of the 48h treatment period are presented as means of each time point ± standard error of the mean (SEM) and analyzed using GraphPad Prism. Paired t-tests were performed and means were considered significantly different when p values were <0.05.

Analysis of the 7 day data were performed by ANOVA repeated measurements using SigmaStat statistical software package and differences were considered significant when p values were <0.05.

### Urinary biomarkers during the 48 hr treatment period

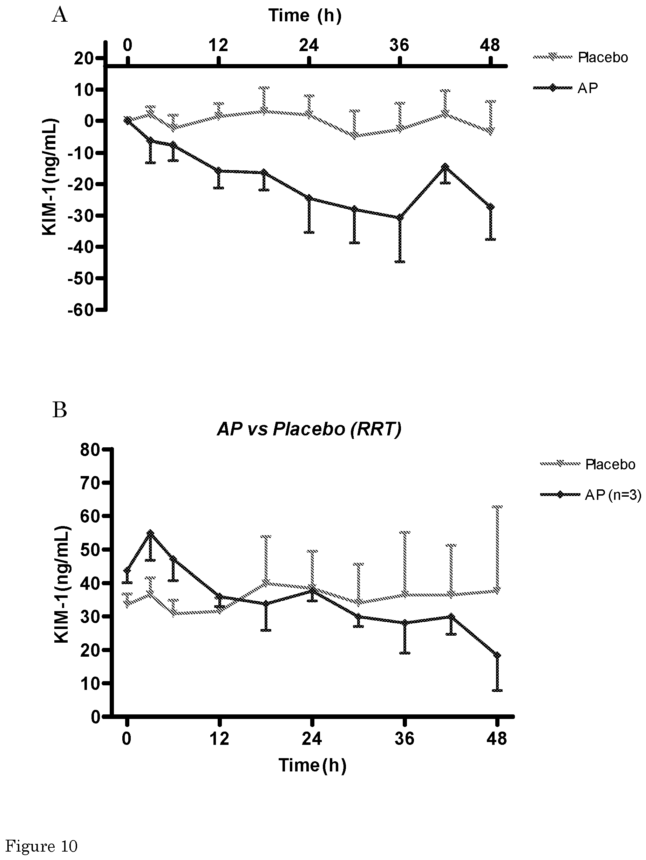

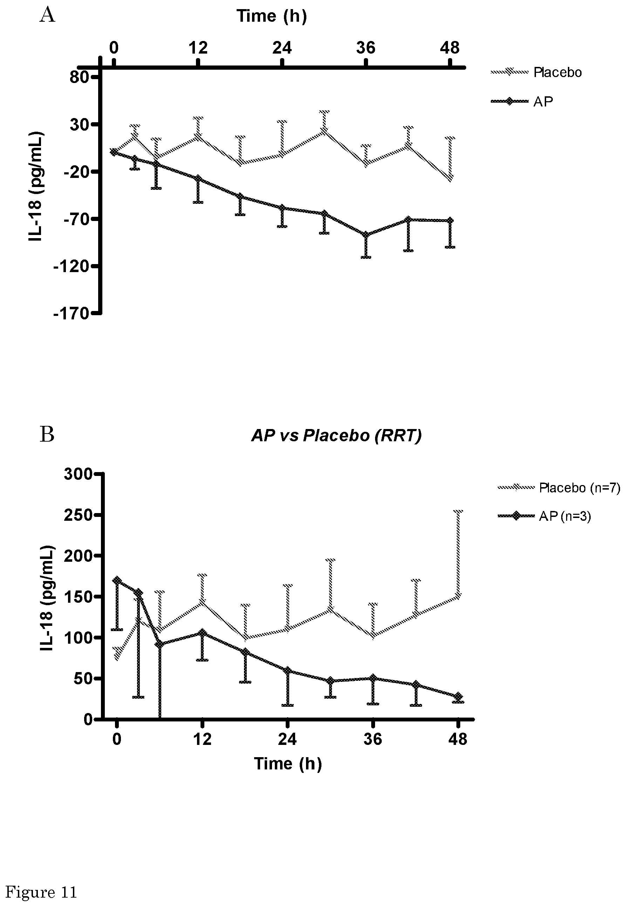

All analyses were performed on baseline corrected values. Statistically significant (paired t-test) treatment effects in favor of BIAP were found for KIM-1 (p=0.0002) (fig. 10) and IL18 (p=0.0027) (fig. 11) during the first 48h of treatment. No statistically significant differences between placebo and treatment groups were found for NGAL or IL-6 (data not shown).

### Analysis of urine and serum biomarkers during 7 days after start of treatment.

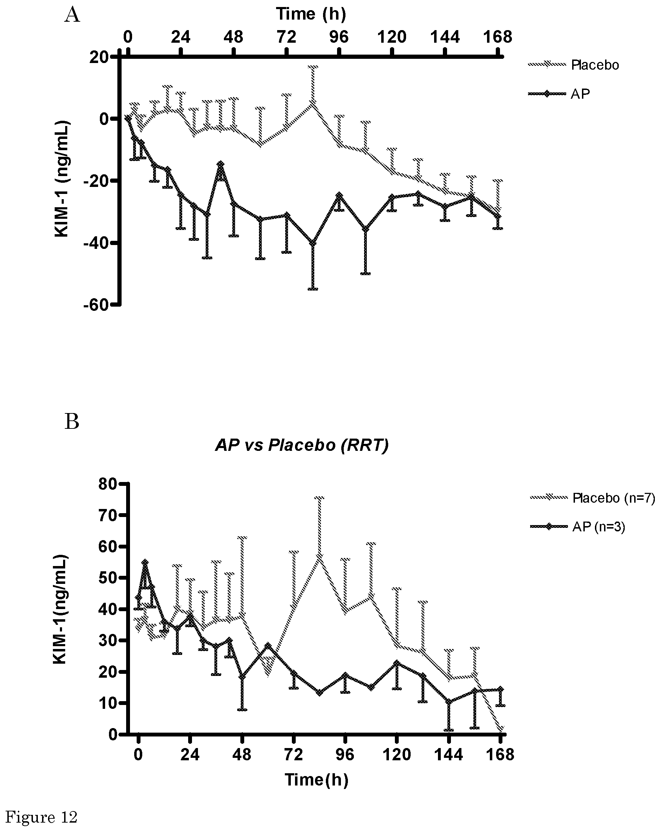

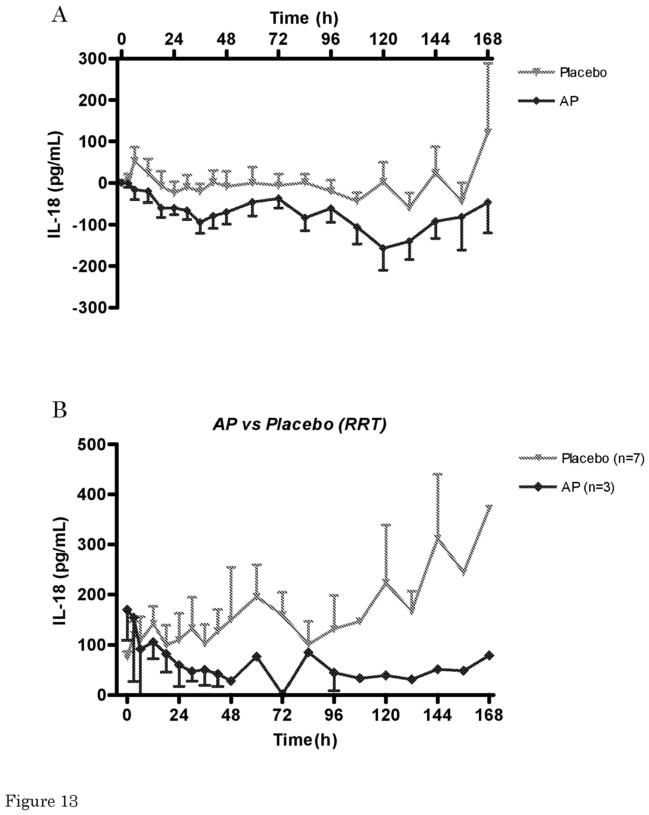

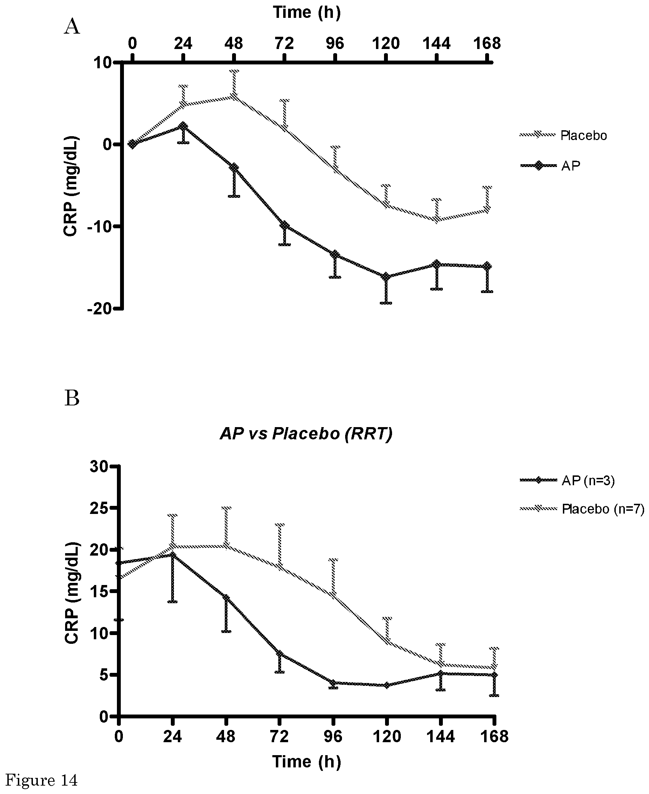

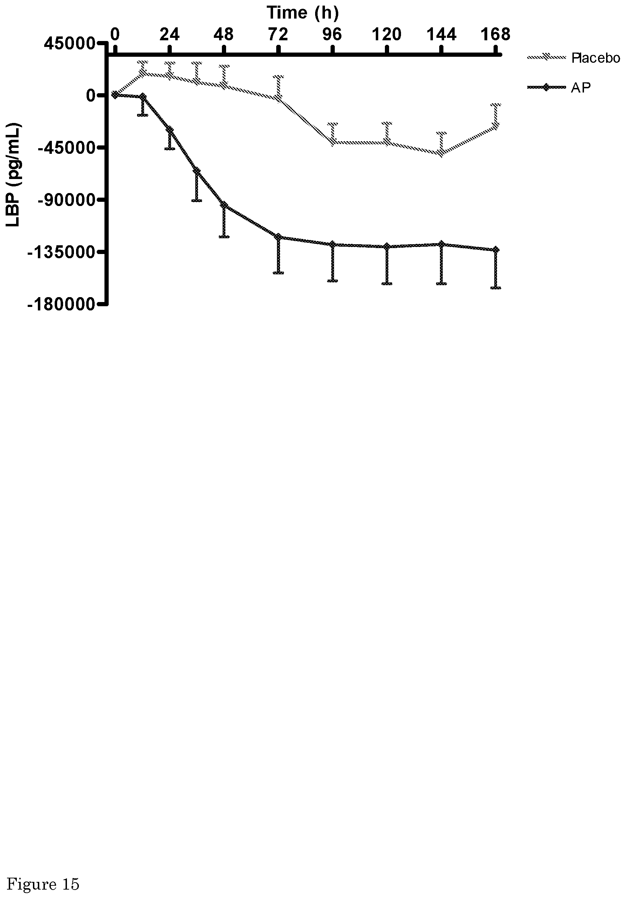

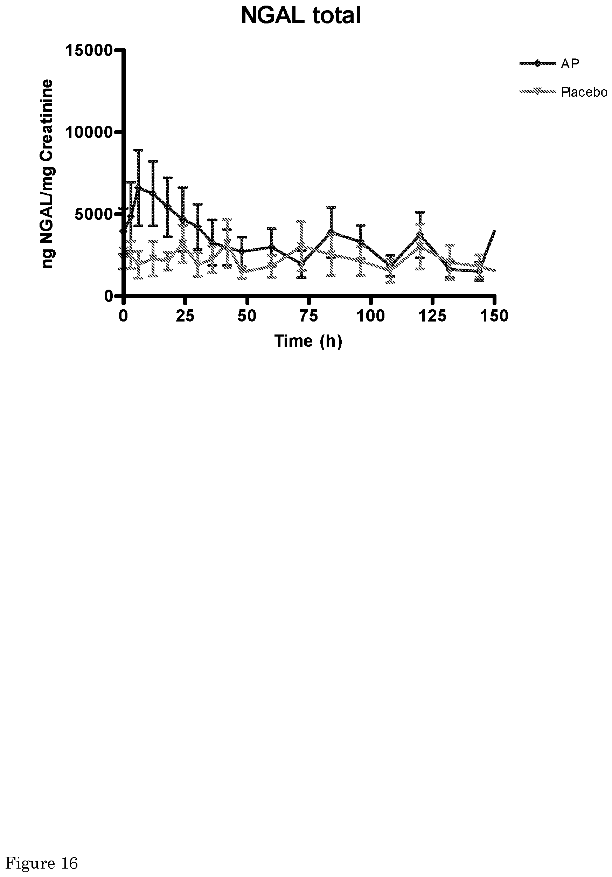

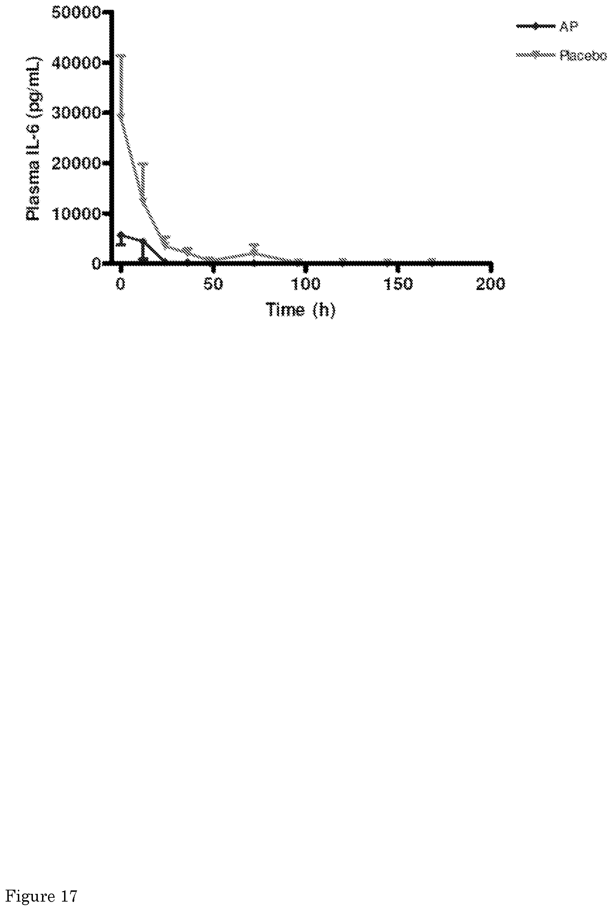

Levels of KIM-1, IL-18 and NGAL (in urine) and CRP, LBP and IL-6 (in serum) were determined. All analyses were performed on baseline corrected values (Figs. 12-15). Statistically significant (ANOVA repeated measurements) treatment effects in favor of BIAP were found for KIM-1, IL18, CRP and LBP (p<0.0001) during 7 days after the start of treatment. Differences, albeit not statistically significant, between placebo and treatment groups were found for NGAL and IL-6 (Figures 16 and 17).

### References

Levey AS, Green T, Kusek JW, Beck GJ, MDRD Study Group. A simplified equation to predict glomerular filtration rate from serum creatinine. J Am Soc Nephrol. 2000;11:A0828.

## Claims

1. Alkaline phosphatase for use in a method for preventing and/or treating atrial fibrillation, wherein said atrial fibrillation is due to reduced renal function.

2. Alkaline phosphatase for use according to claim 1, wherein said atrial fibrillation is due to decreased renal creatinine clearance.

3. Alkaline phosphatase for use according to claim 1 or 2, wherein (co)medication for the treatment of atrial fibrillation is decreased.

4. Alkaline phosphatase for use according to any one of claims 1-3, wherein atrial fibrillation is prevented or treated in an intensive care unit patient.

## Patentansprüche

1. Alkalische Phosphatase zur Verwendung in einem Verfahren zur Vorbeugung und/oder Behandlung von Vorhofflimmern, wobei das Vorhofflimmern auf eingeschränkter Nierenfunktion beruht.

2. Alkalische Phosphatase zur Verwendung nach Anspruch 1, wobei das Vorhofflimmern auf verminderter renaler Kreatinin-Clearance beruht.

3. Alkalische Phosphatase zur Verwendung nach Anspruch 1 oder 2, wobei (Co)Medikation zur Behandlung von Vorhofflimmern vermindert wird.

4. Alkalische Phosphatase zur Verwendung nach einem der Ansprüche 1-3, wobei Vorhofflimmern bei einem Patienten auf Intensivstation verhindert oder behandelt wird.

## Revendications

1. Phosphatase alcaline pour utilisation dans un procédé de prévention et/ou de traitement de la fibrillation auriculaire, pour laquelle ladite fibrillation auriculaire est due à une fonction rénale réduite.

2. Phosphatase alcaline pour l'utilisation selon la revendication 1, dans laquelle ladite fibrillation auriculaire est due à une clairance rénale réduite de la créatinine.

3. Phosphatase alcaline pour l'utilisation selon la revendication 1 ou 2, dans laquelle on a une diminution de la (co)médication destinée au traitement de la fibrillation auriculaire.

4. Phosphatase alcaline pour l'utilisation selon l'une quelconque des revendications 1 à 3, dans laquelle la fibrillation auriculaire est prévenue ou traitée chez un patient en unité de soins intensifs.
